(19) Europäisches Patentamt
European Patent Office
Office européen des brevets

(11) EP 4 809 984 A1

(12) **EUROPEAN PATENT APPLICATION**
published in accordance with Art. 153(4) EPC

(43) Date of publication:
**23.09.2026 Bulletin 2026/39**

(21) Application number: **24891421.0**

(22) Date of filing: **13.11.2024**

(51) International Patent Classification (IPC):
***A61F 2/16** (2006.01)* ***G02C 7/00** (2006.01)*
***G02C 7/02** (2006.01)*

(52) Cooperative Patent Classification (CPC):
**A61F 2/16; G02C 7/00; G02C 7/02**

(86) International application number:
**PCT/JP2024/040261**

(87) International publication number:
**WO 2025/105385 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.11.2023 JP 2023193932**

(71) Applicant: **Hoya Medical Singapore Pte Ltd**
**Chromos 138670 (SG)**

(72) Inventor: **SANGER Demas**
**Tokyo 160-8347 (JP)**

(74) Representative: **Eisenführ Speiser**
**Patentanwälte Rechtsanwälte PartGmbB**
**Postfach 10 60 78**
**28060 Bremen (DE)**

# (54) ASPHERICAL INTRAOCULAR LENS, DESIGN METHOD FOR SAME, AND PRODUCTION METHOD FOR SAME

(57) An intraocular lens in which change in image quality is robust against changes in decentration, tilt, and pupil size, and a related technology thereof are provided. Provided are an intraocular lens and a related technology thereof including a power profile V that is included within a region of a set of power profiles obtained by respective combinations of power profiles Va and Vb when an intersection point of the power profile Va for a first zone created by adding values obtained by multiplying values obtained by subtracting vertical axis values in respective horizontal axis values of an aspherical reference power profile W from a base power by a predetermined ratio $\alpha$ ($\alpha$ is 10% or more and 50% or less) to the vertical axis values in the respective horizontal axis values of the aspherical reference power profile W, and the power profile Vb for a second zone created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W is a position ra in a horizontal axis.

FIG. 2

100%
50%
10%
Va(I50)
Vb(O50)
Va (I10)
Vb (O10)
50%
10%
100%
Dioptric power (D)
Spheric
Zero aberration
Aspheric (SA -0.27)
New IOL
Radius (mm)
ral
rah
21
20
19
18
17
16
0
0.5
1
1.5
2
2.5
3



Processed by Luminess, 75001 PARIS (FR)

## Description

### Technical Field

The present invention relates to an aspherical intraocular lens, a method for designing the same, and a method for manufacturing the same.

### Background Art

Intraocular lenses are known as those that play a role in correcting vision after crystalline lenses opacified by cataracts are extracted. For example, when a crystalline lens becomes opaque due to a cataract, vision is restored by a surgical procedure in which an artificial intraocular lens is inserted into a crystalline lens capsule in place of the opaque crystalline lens.

Paragraph [0007] of Patent Literature 1 describes the problem of how to obtain an intraocular lens that causes little reduction in contrast even when the optical axis of the intraocular lens is misaligned with the optical axis of the eyeball when inserted into the eye, while maintaining the advantage of being able to clearly see an image of the conventional aberration-reducing intraocular lens.

In order to solve the problem, [claim 1] and the like of Patent Literature 1 indicate that when the power distribution set to cancel out the spherical aberration of the cornea when the intraocular lens is inserted into an eye is defined as a reference power distribution, the intraocular lens decreases reduction in contrast that occurs when the optical axis of the intraocular lens inserted into the eye deviates from the optical axis of the eyeball, by having a power distribution that includes at least one positive power shift region that is a region having a power larger than the power represented by the reference power distribution, and at least one negative power shift region that is a region having a power smaller than the power represented by the reference power distribution, in a region in a vicinity of the center of the intraocular lens.

Other intraocular lenses as follows are also known.

For example, Patent Literature 2 and Patent Literature 3 propose intraocular lenses that do not add new spherical aberrations to the existing spherical aberrations of corneas (claim 1 of Patent Literature 2, claim 1 of Patent Literature 3). In these intraocular lenses, power values are constant in all the regions of the optical portions. Since no spherical aberration is added to the ocular surface optical system, there is no effect of image quality degradation due to lens decentration and tilt. Paragraph [0005] of Patent Literature 2 proposes an aspherical intraocular lens in which an amount of negative spherical aberration is smaller than the amount required to offset the positive spherical aberration of the cornea.

Patent Literature 4 proposes an aspherical intraocular lens that reduces the spherical aberration of the cornea of an average human eye in order to minimize the spherical aberration of the optical eye system (claims 1, 13, and the like of Patent Literature 4). The power value of such an aspherical intraocular lens decreases as the radius of the lens increases. In order for this lens to provide high image quality, the decentration and tilt of the lens need to be kept low in a patient having an aphakic eye.

Patent Literature 5 proposes an aspherical intraocular lens that can provide a balance between the contrast of an image and depth of focus within an allowable range, especially under large pupil (for example, 4.5 to 5 mm) conditions (claim 1 of Patent Literature 5).

Patent Literature 6 proposes an aspherical intraocular lens that provides minimum sensitivity of optical performance under the occurrence of lens concentration and lens tilt. The power value of the lens decreases first, and then increases as the radius of the lens increases (black circle in Figure 6 of Patent Literature 6).

### Citation List

#### Patent Literature

[Patent Literature 1] Japanese Patent Laid-Open No. 2007-330478
[Patent Literature 2] US2005/0203619
[Patent Literature 3] WO2004/090611
[Patent Literature 4] US2004/0088050
[Patent Literature 5] WO2006/060477
[Patent Literature 6] WO2007/128423

### Summary of Invention

Technical Problem

It is also assumed that after an intraocular lens is inserted into an eye, an optical axis center of the intraocular lens is displaced from the cornea center (decentration), and an optical axis of the intraocular lens tilts from the thickness direction of the cornea (eye axis direction) (tilting).

In addition, it is also assumed that a wearer having an intraocular lens inserted in the eye will work outdoors where there is a large amount of light, or work indoors (or a dark place) where there is a relatively small amount of light. In other words, it is also assumed that the pupil size of the wearer will change.

An object of the present invention is to provide an intraocular lens in which a change in image quality is robust against changes in the above-described decentration, tilt, and pupil size while the image quality remains good, and a related technology of the same.

Solution to Problem

A first aspect is

an intraocular lens including at least two zones for vision correction that are concentric with a lens center O to which a predetermined base power is set and adjacent to each other,
wherein when the zones for vision correction are set as, in order from a first zone including the lens center O outward in a radial direction, the first zone, and a second zone surrounding the first zone, and
a position of a first boundary between the first zone and the second zone when seen in the radial direction from the lens center O is r1, in a power profile in which a position when seen in the radial direction from the lens center O is set as a horizontal axis (unit: mm), and power is set as a vertical axis (unit: D (diopter)), and
when a power profile in a virtual aspherical lens that has a base power in the lens center O and completely cancels positive longitudinal spherical aberration provided by a cornea is an aspherical reference power profile W,
the intraocular lens includes:

a power profile V that is included within a region of a set of power profiles obtained by respective combinations of power profiles Va and Vb when an intersection point of
the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W, and
the power profile Vb for the second zone created by adding values obtained by multiplying values obtained by subtracting the horizontal axis values in the respective vertical axis values of the aspherical reference power profile W from a maximum value rmax of the horizontal axis of the aspherical reference power profile W by a predetermined ratio $\beta$ ($\beta$ is 10% or more and 50% or less) to the horizontal axis values in the respective vertical axis values of the aspherical reference power profile W

is a position ra in a horizontal axis.

A second aspect is
the intraocular lens according to the first aspect, wherein rl is a value within a range of 1.5 mm or more and 2.3 mm or less.

A third aspect is
the intraocular lens according to the first aspect, wherein the position r1 has a positive maximum value among values obtained by subtracting the vertical axis values of the aspherical reference power profile W from vertical axis values of the power profile V, in respective horizontal axis values of the power profile V.

A fourth aspect is
the intraocular lens according to the first aspect, wherein a maximum value rmax of a horizontal axis of the power profile V is a value within a range of 2.5 mm or more and 3.5 mm or less.

A fifth aspect is

the intraocular lens according to the first aspect, including
one or more additional regions from the second zone outward in the radial direction,
wherein the additional regions surround the second zone, and
in respective horizontal axis values of a power profile Vadd in at least one region of the additional regions, values obtained by subtracting the vertical axis values of the aspherical reference power profile W from vertical axis values of the power profile Vadd are less than $\pm$0.30 D.

A sixth aspect is
the intraocular lens according to the first aspect, wherein the power profile V is expressed by a polynomial expression.
A seventh aspect is

the intraocular lens according to the first aspect, including
one or more additional regions from the second zone outward in the radial direction,
wherein the additional regions surround the second zone, and
the additional regions include a function of refracting incident light beams onto a retina.

An eighth aspect is

an intraocular lens including at least two zones for vision correction that are concentric with a lens center O to which a predetermined base power is set and adjacent to each other,
wherein when the zones for vision correction are set as, in order from a first zone including the lens center O outward in a radial direction, the first zone, and a second zone surrounding the first zone, and
a position of a first boundary between the first zone and the second zone when seen in the radial direction from the lens center O is r1, in a power profile in which a position seen in the radial direction from the lens center O is set as a horizontal axis (unit: mm), and a power is set as a vertical axis (unit: D (diopter)), and
when a power profile in a virtual aspherical lens that has a base power in the lens center O and completely cancels positive longitudinal spherical aberration provided by a cornea is an aspherical reference power profile W,
in the first zone, an average value of vertical axis values of a power profile V1 for the first zone is larger than an average value of vertical axis values of the aspherical reference power profile W,
in the second zone, an average value of vertical axis values of a power profile V2 for the second zone is larger than the average value of the vertical axis values of the aspherical reference power profile W,
in the power profile V1 for the first zone, with increase in a horizontal axis value, a value obtained by subtracting a vertical axis value of the aspherical reference power profile W from a vertical axis value of the power profile V1 continuously increases in each of horizontal axis values,
in the power profile V2 for the second zone, with increase in a horizontal axis value, a value obtained by subtracting the vertical axis value of the aspherical reference power profile W from a vertical axis value of the power profile V2 continuously decreases in each of horizontal axis values,
in a power profile V, the position r1 has a positive maximum value among values obtained by subtracting the vertical axis values of the aspherical reference power profile W from the vertical axis values of the power profile V2, and
a value obtained by subtracting a vertical axis value of the aspherical reference power profile W from a vertical axis value in a maximum value rmax of a horizontal axis of the power profile V is less than 0.25 D.

A ninth aspect is
the intraocular lens according to the eighth aspect, wherein an absolute value of an average value of a slope of a tangent line of the power profile V2 in the second zone is equal to or more than three times an absolute value of an average value of a slope of a tangent line of the power profile V1 in the first zone.
A tenth aspect is
the intraocular lens according to the eighth aspect, wherein r1 is a value within a range of 1.5 mm or more and 2.3 mm or less.
An eleventh aspect is
the intraocular lens according to the eighth aspect, wherein the maximum value rmax of the horizontal axis of the power profile V is a value within a range of 2.5 mm or more and 3.5 mm or less.
A twelfth aspect is

the intraocular lens according to the eighth aspect, including the power profile V that is included within a region of a set of power profiles obtained by respective combinations of power profiles Va and Vb, when an intersection point of the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W, and
the power profile Vb for the second zone created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W

is a position ra in the horizontal axis.

A thirteenth aspect is
the intraocular lens according to the eighth aspect including:

one or more additional regions from the second zone outward in the radial direction,
wherein the additional regions surround the second zone,
in respective horizontal axis values of a power profile Vadd in at least one region of the additional regions, values obtained by subtracting the vertical axis values of the aspherical reference power profile W from vertical axis values of the power profile Vadd are less than ±0.30 D.

A fourteenth aspect is
the intraocular lens according to the eighth aspect, wherein the power profile V is expressed by a polynomial expression.

A fifteenth aspect is

the intraocular lens according to the eighth aspect including one or more additional regions from the second zone outward in the radial direction,
wherein the additional regions surround the second zone, and
the additional regions include a function of refracting incident light beams onto a retina.

A sixteenth aspect is

an intraocular lens including at least two zones for vision correction that are concentric with a lens center O to which a predetermined base power is set and adjacent to each other,
wherein when the zones for vision correction are set as, in order from a first zone including the lens center O outward in a radial direction, the first zone, a middle zone surrounding the first zone, and a second zone surrounding the middle zone, and
in a power profile in which a position when seen in the radial direction from the lens center O is set as a horizontal axis (unit: mm), and a power is set as a vertical axis (unit: D (diopter)),
a power profile in a virtual aspherical lens that has a base power in the lens center O and completely cancels positive longitudinal spherical aberration provided by a cornea is an aspherical reference power profile W,
the intraocular lens includes a power profile V that is included within a region of a set of power profiles obtained by respective combinations of power profiles Va, Vmid and Vb, configured by
the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W,
the power profile Vb for the second zone created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W, and
the power profile Vmid for the middle zone in which an average power is smaller than an average power in the first zone, and larger than an average power in the second zone.

A seventeenth aspect is
the intraocular lens according to the sixteenth aspect, wherein an absolute value of an average value of a slope of a tangent line of the power profile Vmid in the middle zone is larger than an absolute value of an average value of a slope of a tangent line of a power profile V1 in the first zone, and is smaller than an absolute value of an average value of a slope of a tangent line of a power profile V2 in the second zone.

An eighteenth aspect is

the intraocular lens according to the sixteenth aspect,
wherein in respective horizontal axis values of the power profile Vmid in the middle zone, an average value of values obtained by subtracting the vertical axis values of the aspherical reference power profile W from vertical axis values of the power profile Vmid is
larger than an average value of values obtained by subtracting the vertical axis values of the aspherical reference power profile W from vertical axis values of a power profile V1 in respective horizontal axis values of the power profile V1 in the first zone, and

larger than an average value of values obtained by subtracting the vertical axis values of the aspherical reference power profile W from vertical axis values of a power profile V2 in respective horizontal axis values of the power profile V2 in the second zone.

A nineteenth aspect is
the intraocular lens according to the sixteenth aspect, wherein in the middle zone, horizontal axis values are included within a range of 1.3 mm or more and 2.5 mm or less.

A twentieth aspect is
the intraocular lens according to the sixteenth aspect, wherein a maximum value rmax of a horizontal axis of the power profile V is a value within a range of 2.5 mm or more and 3.5 mm or less.

A twenty-first aspect is
the intraocular lens according to the sixteenth aspect, wherein an absolute value of an average value of a slope of a tangent line of a power profile V2 in the second zone is equal to or larger than three times an absolute value of an average value of a slope of a tangent line of a power profile V1 in the first zone.

A twenty-second aspect is

the intraocular lens according to the sixteenth aspect including one or more additional regions outward in the radial direction from the second zone,
wherein the additional regions surround the second zone, and
in respective horizontal axis values of a power profile Vadd in at least one region of the additional regions, values obtained by subtracting the vertical axis values of the aspherical reference power profile W from vertical axis values of the power profile Vadd are less than ±0.30 D.

A twenty-third aspect is
the intraocular lens according to the sixteenth aspect, wherein the power profile V is expressed by a polynomial expression.

A twenty-fourth aspect is

the intraocular lens according to the sixteenth aspect, including one or more additional regions outward in the radial direction from the second zone,
wherein the additional regions surround the second zone, and
the additional regions include a function of refracting incident light beams onto a retina.

A twenty-fifth aspect is

an intraocular lens including at least two zones for vision correction that are concentrical with a lens center O to which a predetermined base power is set and adjacent to each other,
wherein when the zones for vision correction are set as, in order from a first zone including the lens center O outward in a radial direction, the first zone, and a second zone surrounding the first zone, and
a power profile in a virtual aspherical lens that has a base power in the lens center O and completely cancels positive longitudinal spherical aberration provided by a cornea is an aspherical reference power profile W, and
when a position of a first boundary between the first zone and the second zone seen in the radial direction from the lens center O is r1, in a total power profile in which a position when seen in the radial direction from the lens center O is set as a horizontal axis (unit: mm), a total power T when refractive power of the cornea and power of the intraocular lens are added up is set as a vertical axis (unit: D (diopter)),
the intraocular lens includes a total power profile TV that is included within a region of a set of total power profiles obtained by respective combinations of total power profiles TVa and TVb, when an intersection point of
the total power profile TVa for the first zone created by adding values obtained by multiplying values obtained by subtracting vertical axis values in respective horizontal axis values of an aspherical reference total power profile TW in which the refractive power of the cornea is added from the base power by a predetermined ratio $\alpha$ ($\alpha$ is 10% or more and 50% or less) to the vertical axis values in the respective horizontal axis values of the aspherical reference total power profile TW, and
the total power profile TVb for the second zone created by adding values obtained by multiplying values obtained by subtracting the horizontal axis values in the respective vertical axis values of the aspherical reference total power profile TW in which the refractive power of the cornea is added from a maximum value rmax in a horizontal axis of the aspherical reference total power profile TW by a predetermined ratio $\beta$ ($\beta$ is 10% or more and 50% or less) to the horizontal axis values in the respective vertical axis values of the aspherical reference total power profile TW

is a position ra in a horizontal axis.

A twenty-sixth aspect is

the intraocular lens according to the twenty-fifth aspect, wherein
in a total power profile TV1 for the first zone, with increase in a horizontal axis value, a vertical axis value continuously increases, and
in a total power profile TV2 for the second zone, with increase in the horizontal axis value, the vertical axis value continuously decreases.

A twenty-seventh aspect is

the intraocular lens according to the twenty-fifth aspect,
wherein an absolute value of an average value of a slope of a tangent line of a total power profile TV1 in a vicinity of an origin in the first zone is smaller than an absolute value of an average value of a slope of a tangent line of the total power profile TV1 in a vicinity of a middle position in the first zone,
the absolute value of the average value of the slope of the tangent line of the total power profile TV1 in the vicinity of the middle position in the first zone is larger than an absolute value of an average value of a slope of a tangent line of the total power profile TV1 in a vicinity of the position r1 in the first zone, and
the absolute value of the average value of the slope of the tangent line of the total power profile TV1 in the vicinity of the position r1 in the first zone is smaller than an absolute value of an average value of a slope of a tangent line of a total power profile TV2 in a vicinity of a middle position in the second zone.

A twenty-eighth aspect is

the intraocular lens according to the twenty-fifth aspect,
wherein in the total power profile TV, a value obtained by subtracting a vertical axis value in r=0 from a vertical axis value in the position r1 is a positive maximum value, and
r1 is a value within a range of 1.5 mm or more and 2.3 mm or less.

A twenty-ninth aspect is
the intraocular lens according to the twenty-fifth aspect, wherein a value obtained by subtracting a vertical axis value in r=0 from a vertical axis value in a maximum value rmax of a horizontal axis of the total power profile TV is less than ±0.30 D.

A thirtieth aspect is
the intraocular lens according to the twenty-fifth aspect, wherein a maximum value rmax of a horizontal axis of the total power profile TV is a value within a range of 2.5 mm or more and 3.5 mm or less.

A thirty-first aspect is

the intraocular lens according to the twenty-fifth aspect, including one or more additional regions from the second zone outward in the radial direction,
wherein the additional regions surround the second zone, and
the additional regions include a function of refracting incident light beams onto a retina.

A thirty-second aspect is

an intraocular lens including at least two zones for vision correction that are concentric with a lens center O to which a predetermined base power is set and adjacent to each other,
wherein when the zones for vision correction are set as, in order from a first zone including the lens center O outward in a radial direction, the first zone, and a second zone surrounding the first zone,
in a power profile in which a position when seen in the radial direction from the lens center O is set as a horizontal axis (unit: mm), and a power is set as a vertical axis (unit: D (diopter)),
a power profile in a virtual aspherical lens that has a base power in the lens center O and completely cancels positive longitudinal spherical aberration provided by a cornea is an aspherical reference power profile W,
a power profile created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W is a power profile Va for the first zone, and
a power profile created by adding values obtained by multiplying values obtained by subtracting the horizontal axis values in the respective vertical axis values of the aspherical reference power profile W from a maximum value rmax of

a horizontal axis of the aspherical reference power profile W by a predetermined ratio β (β is 10% or more and 50% or less) to the horizontal axis values in the respective vertical axis values of the aspherical reference power profile W is a power profile Vb for the second zone,
the first zone includes an inner first region including a power profile in which a positive power is added to the power profile Va for the first zone,
the second zone includes the power profile Vb, and
an area of the inner first region is less than 50% of a total area of the first zone.

A thirty-third aspect is

the intraocular lens according to the thirty-second aspect,
wherein the first zone incudes the inner first region, and an outer first region surrounding the inner first region, when seen in the radial direction from the lens center O, and
the outer first region includes the power profile Va, and is in contact with the second zone in a position of an intersection point of the power profiles Va and Vb.

A thirty-fourth aspect is
the intraocular lens according to the thirty-second aspect, wherein an anterior surface, a posterior surface, or both of them of a lens body of the intraocular lens having two surfaces facing each other is or are equipped with the inner first region.
A thirty-fifth aspect is
the intraocular lens according to the thirty-second aspect, wherein the first zone includes a region that is other than the inner first region, and has an average power equal to an average power of the power profile Va, and an average power of the inner first region is larger than the average power of the region other than the inner first region by 0.75 to 4.0 D.
A thirty-sixth aspect is
the intraocular lens according to the thirty-third aspect, wherein the first zone includes an outer transition region for connecting the inner first region to the outer first region.
A thirty-seventh aspect is

the intraocular lens according to the thirty-second aspect,
wherein the inner first region includes a constant power addition region including a power profile in which a positive constant power is added to the power profile Va for the first zone, and
when seen in the radial direction from the lens center O, a range in the radial direction of the constant power addition region is 33 to 67% of a range in the radial direction of the inner first region.

A thirty-eighth aspect is
the intraocular lens according to the thirty-second aspect, wherein the inner first region includes the lens center O.
A thirty-ninth aspect is

the intraocular lens according to the thirty-third aspect,
wherein the first zone includes an innermost region including the power profile Va for the first zone and including the lens center O, and
the inner first region surrounds the innermost region.

A fortieth aspect is
the intraocular lens according to the thirty-ninth aspect, wherein the first zone includes an inner transition region for connecting the inner first region to the innermost region.
A forty-first aspect is
the intraocular lens according to the thirty-second aspect, wherein an anterior surface, a posterior surface, or both of them of a lens body of the intraocular lens having two surfaces facing each other is or are aspherical.
A forty-second aspect is
the intraocular lens according to the thirty-second aspect, wherein an anterior surface, a posterior surface, or both of them of a lens body of the intraocular lens having two surfaces facing each other includes or include a toric surface including a cylinder power for astigmatism correction for an aphakic patient.
A surface of the lens lathe-turned (formed) into an aspherical surface to realize (create) the power profile taught by the contents (specification of the present application) laid-open by the present application may be only an anterior surface, only a posterior surface or the anterior surface and the posterior surface (both the surfaces).
A forty-third aspect is
the intraocular lens according to any one of the first to the forty-second aspects, including at least any one of silicone,

hydrophobic acrylic resin, hydrophilic acrylic resin, hydrogel, PMMA, PMMA copolymer, and collagen-containing HEMA (hydroxyethyl methacrylate) copolymer.

A forty-fourth aspect is

a method for designing an intraocular lens designing the intraocular lens according to any one of the first to the forty-second aspects.

A forty-fifth aspect is

a method for manufacturing an intraocular lens manufacturing the intraocular lens designed by the method for designing an intraocular lens according to the forty-fourth aspect by at least any one of lathe-turning, molding, and 3D printing.

Further, the other aspects of the present invention are enumerated as follows.

An area ratio of the first zone and the second zone in plan view may be set to 25:75 to 75:25.

It is preferable that in the first zone, the power continuously decrease.

It is preferable that in the second zone, the power continuously decrease.

It is preferable that between the lens center O to the position r1 of the first boundary, the total power T at the time of adding up the refractive power of the cornea and the power of the intraocular lens continuously increase.

The technical idea of the present invention can also be applied to the method for designing an intraocular lens or the method for manufacturing an intraocular lens.

## Advantageous Effects of Invention

According to the present invention, the change in image quality becomes robust against the changes in the above-described decentration, tilt and pupil size, while the image quality remains good.

## Brief Description of Drawings

[Figure 1] Figure 1 is a schematic plan view showing an intraocular lens of an embodiment of the present invention.

[Figure 2] Figure 2 is a power profile in which a position seen in a radial direction from a lens center O is set as a horizontal axis (unit: mm), and power is set as a vertical axis (unit: D (diopter)).

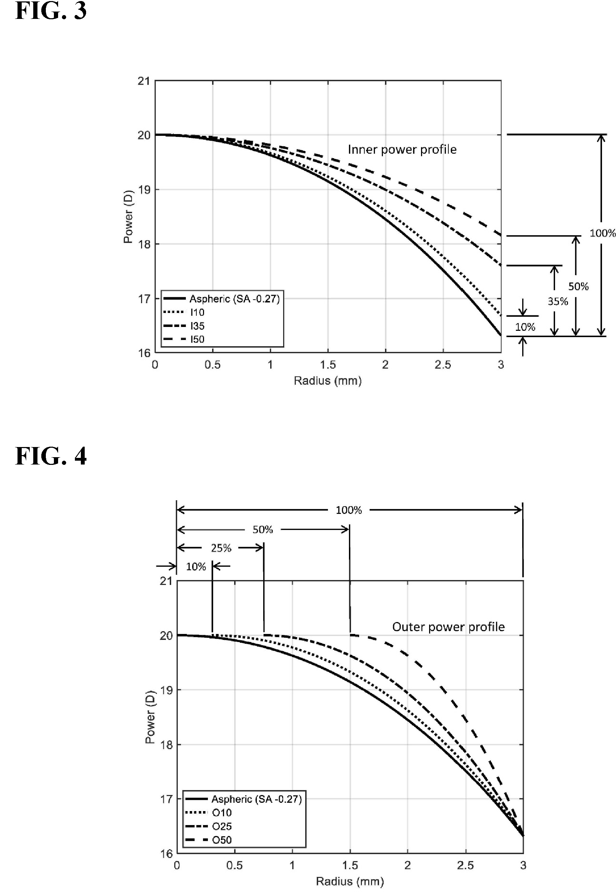

[Figure 3] Figure 3 is a power profile showing refractive power (vertical axis) provided by an optical portion of the intraocular lens with respect to a distance (horizontal axis) from the lens center O, which is a diagram showing a power profile Va created by adding values obtained by multiplying values obtained by subtracting vertical axis values in respective horizontal axis values of an aspherical reference power profile W from a base power by a predetermined ratio $\alpha$ ($\alpha$ is 10% that is I10, 35% that is I35, and 50% that is I50, of values 10% or more and 50% or less) to the vertical axis values in the respective horizontal axis values of the aspherical reference power profile W.

[Figure 4] Figure 4 is a power profile showing refractive power (vertical axis) provided by the optical portion of the intraocular lens with respect to the distance (horizontal axis) from the lens center O, which is a diagram showing a power profile Vb created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% that is O10, 25% that is O25, and 50% that is O50 of values 10% or more and 50% or less) to the horizontal axis values in the respective vertical axis values of the aspherical reference power profile W.

[Figure 5] Figure 5 is an explanatory diagram at a time of I35 in Figure 3 and O25 in Figure 4 being superimposed on each other.

[Figure 6] Figure 6 is a diagram showing a power profile V created by patching together a power profile Va (I35) for a first zone and a power profile Vb (O25) for a second zone in Figure 5.

[Figure 7] Figure 7 is a diagram showing I10-O10, 140-020, and 150-050.

[Figure 8] Figure 8 is a diagram showing a region (hatched portion) configured by a power profile when an intersection point of the power profile Va for the first zone ($\alpha$ is 10% or more and 50% or less) and the power profile Vb for the second zone ($\beta$ is 10% or more and 50% or less) is a position ra in the horizontal axis.

[Figure 9] Figure 9 is a diagram showing a power profile V (solid line) in the case of not adopting what is created by directly patching together mutually different power profiles, but adopting a profile following what is created by patching together the mutually different power profiles, in [Others] in Description of Embodiments.

[Figure 10] Figure 10 is a diagram showing a power profile V (solid line, 135-025, an optical portion diameter 6.50 mm) in the case of being provided with an additional region.

[Figure 11] Figure 11 is a diagram showing I10-O10, and I50-O50, which is a diagram in which absolute values of an average value of a slope of a tangent line of a power profile V1 in the first zone and an average value of a slope of a tangent line of a power profile V2 in the second zone are both written with respect to each of the power profiles V.

[Figure 12A] Figure 12A is a diagram showing a power profile V (solid line, I35-O25) in the case of being provided with a middle zone.

[Figure 12B] Figure 12B is a diagram in the case of the middle zone in Figure 12A being expanded.

[Figure 12C] Figure 12C is a diagram showing a power profile V (solid line, I40-O40) in the case of being provided with a middle zone.

[Figure 13] Figure 13 is a table showing conditions adopted in a total power profile according to the embodiment of the present invention.

[Figure 14] Figure 14 is a total power profile TV showing a total power (total power) T (vertical axis) at a time of adding up refractive power of a cornea and power of the intraocular lens, with respect to a distance (horizontal axis) from the lens center O.

[Figure 15] Figure 15 is a diagram showing the total power profile TV in the case where a vertical axis value is matched with a vertical axis value at a time of a horizontal axis value of zero in a horizontal axis value rmax of 3.0 mm.

[Figure 16] Figure 16 is a diagram showing a region (hatched portion) configured by total power profiles when an intersection point of a total power profile TVa for the first zone ($\alpha$ is 10% or more and 50% or less) and a total power profile TVb for the second zone ($\beta$ is 10% or more and 50% or less) is a position ra in the horizontal axis.

[Figure 17A] Figure 17A is a plot showing an MTF value (vertical axis) indicating contrast with respect to an object distance (distance) from a corneal apex (horizontal axis) (aperture diameter (pupil size) set to 3.0 mm, and no decentration or tilt). The MTF value shown in Figures 17 to 30 is an MTF value when a spatial frequency is 100 lines/mm. The spatial frequency of the MTF value (vertical axis) is 100 lines/mm.

[Figure 17B] Figure 17B is a diagram in which the aperture diameter is set to 3.5 mm in Figure 17A.

[Figure 17C] Figure 17C is a diagram in which the aperture diameter is set to 4.0 mm in Figure 17A.

[Figure 17D] Figure 17D is a diagram in which the aperture diameter is set to 4.5 mm in Figure 17A.

[Figure 18A] Figure 18A is a plot showing the MTF value (vertical axis) indicating contrast with respect to the object distance (distance) from the corneal apex (horizontal axis) (aperture diameter (pupil size) set to 3.0 mm, decentration 0.3 mm, and no tilt).

[Figure 18B] Figure 18B is a diagram in which the aperture diameter is set to 3.5 mm in Figure 18A.

[Figure 18C] Figure 18C is a diagram in which the aperture diameter is set to 4.0 mm in Figure 18A.

[Figure 18D] Figure 18D is a diagram in which the aperture diameter is set to 4.5 mm in Figure 18A.

[Figure 19A] Figure 19A is a plot showing the MTF value (vertical axis) indicating contrast with respect to the object distance (distance) (horizontal axis) from the corneal apex (aperture diameter (pupil size) set to 3.0 mm, decentration 0.5 mm, and no tilt).

[Figure 19B] Figure 19B is a diagram in which the aperture diameter is set to 3.5 mm in Figure 19A.

[Figure 19C] Figure 19C is a diagram in which the aperture diameter is set to 4.0 mm in Figure 19A.

[Figure 19D] Figure 19D is a diagram in which the aperture diameter is set to 4.5 mm in Figure 19A.

[Figure 20A] Figure 20A is a plot showing the MTF value (vertical axis) indicating contrast with respect to the object distance (distance) (horizontal axis) from the corneal apex (aperture diameter (pupil size) set to 3.0 mm, no decentration, and tilt 3 degrees).

[Figure 20B] Figure 20B is a diagram in which the aperture diameter is set to 3.5 mm in Figure 20A.

[Figure 20C] Figure 20C is a diagram in which the aperture diameter is set to 4.0 mm in Figure 20A.

[Figure 20D] Figure 20D is a diagram in which the aperture diameter is set to 4.5 mm in Figure 20A.

[Figure 21A] Figure 21A is a plot showing the MTF value (vertical axis) indicating contrast with respect to the object distance (distance) (horizontal axis) from the corneal apex (aperture diameter (pupil size) set to 3.0 mm, no decentration, and tilt 5 degrees).

[Figure 21B] Figure 21B is a diagram in which the aperture diameter is set to 3.5 mm in Figure 21A.

[Figure 21C] Figure 21C is a diagram in which the aperture diameter is set to 4.0 mm in Figure 21A.

[Figure 21D] Figure 21D is a diagram in which the aperture diameter is set to 4.5 mm in Figure 21A.

[Figure 22A] Figure 22A is a plot showing the MTF value (vertical axis) indicating contrast with respect to the object distance (distance) (horizontal axis) from the corneal apex (aperture diameter (pupil size) set to 3.0 mm, decentration 0.3 mm, and tilt 3 degrees).

[Figure 22B] Figure 22B is a diagram in which the aperture diameter is set to 3.5 mm in Figure 22A.

[Figure 22C] Figure 22C is a diagram in which the aperture diameter is set to 4.0 mm in Figure 22A.

[Figure 22D] Figure 22D is a diagram in which the aperture diameter is set to 4.5 mm in Figure 22A.

[Figure 23A] Figure 23A is a plot showing the MTF value (vertical axis) indicating contrast with respect to the object distance (distance) (horizontal axis) from the corneal apex (aperture diameter (pupil size) set to 3.0 mm, decentration 0.4 mm, and tilt 4 degrees).

[Figure 23B] Figure 23B is a diagram in which the aperture diameter is set to 3.5 mm in Figure 23A.

[Figure 23C] Figure 23C is a diagram in which the aperture diameter is set to 4.0 mm in Figure 23A.

[Figure 23D] Figure 23D is a diagram in which the aperture diameter is set to 4.5 mm in Figure 23A.

[Figure 24A] Figure 24A is a plot showing the MTF value (vertical axis) indicating contrast with respect to the object distance (distance) (horizontal axis) from the corneal apex (aperture diameter (pupil size) set to 3.0 mm, decentration 0.5 mm, and tilt 5 degrees).

[Figure 24B] Figure 24B is a diagram in which the aperture diameter is set to 3.5 mm in Figure 24A.

[Figure 24C] Figure 24C is a diagram in which the aperture diameter is set to 4.0 mm in Figure 24A.

[Figure 24D] Figure 24D is a diagram in which the aperture diameter is set to 4.5 mm in Figure 24A.

[Figure 25A] Figure 25A is a plot showing an MTF value (vertical axis) indicating contrast with respect to decentration (horizontal axis) (object distance set to 2.0 m, aperture diameter (pupil size) 3.0 mm, and no tilt).

[Figure 25B] Figure 25B is a diagram in which the tilt is set to 3 degrees in Figure 25A.

[Figure 25C] Figure 25C is a diagram in which the tilt is set to 5 degrees in Figure 25A.

[Figure 25D] Figure 25D is a diagram in which the aperture diameter is set to 4.0 mm in Figure 25A, and the tilt is set to zero.

[Figure 25E] Figure 25E is a diagram in which the aperture diameter is set to 4.0 mm, and the tilt is set to 3 degrees in Figure 25A.

[Figure 25F] Figure 25F is a diagram in which the aperture diameter is set to 4.0 mm and the tilt is set to 5 degrees in Figure 25A.

[Figure 26A] Figure 26A is a plot showing the MTF value (vertical axis) indicating contrast with respect to decentration (horizontal axis) (object distance set to 3.0 m, aperture diameter (pupil size) 3.0 mm, and no tilt).

[Figure 26B] Figure 26B is a diagram in which the tilt is set to 3 degrees in Figure 26A.

[Figure 26C] Figure 26C is a diagram in which the tilt is set to 5 degrees in Figure 26A.

[Figure 26D] Figure 26D is a diagram in which the aperture diameter is set to 4.0 mm in Figure 26A, and the tilt is set to zero.

[Figure 26E] Figure 26E is a diagram in which the aperture diameter is set to 4.0 mm, and the tilt is set to 3 degrees in Figure 26A.

[Figure 26F] Figure 26F is a diagram in which the aperture diameter is set to 4.0 mm and the tilt is set to 5 degrees in Figure 26A.

[Figure 27A] Figure 27A is a plot showing the MTF value (vertical axis) indicating contrast with respect to decentration (horizontal axis) (object distance set to 4.0 m, the aperture diameter (pupil size) 3.0 mm, and no tilt).

[Figure 27B] Figure 27B is a diagram in which the tilt is set to 3 degrees in Figure 27A.

[Figure 27C] Figure 27C is a diagram in which the tilt is set to 5 degrees in Figure 27A.

[Figure 27D] Figure 27D is a diagram in which the aperture diameter is set to 4.0 mm in Figure 27A, and the tilt is set to zero.

[Figure 27E] Figure 27E is a diagram in which the aperture diameter is set to 4.0 mm and the tilt is set to 3 degrees in Figure 27A.

[Figure 27F] Figure 27F is a diagram in which the aperture diameter is set to 4.0 mm and the tilt is set to 5 degrees in Figure 27A.

[Figure 28A] Figure 28A is a plot showing an MTF value (vertical axis) indicating contrast with respect to the tilt (horizontal axis) (object distance set to 2.0 m, aperture diameter (pupil size) 3.0 mm, and no decentration).

[Figure 28B] Figure 28B is a diagram in which the decentration is set to 0.3 mm in Figure 28A.

[Figure 28C] Figure 28C is a diagram in which the decentration is set to 0.5 mm in Figure 28A.

[Figure 28D] Figure 28D is a diagram in which the aperture diameter is set to 4.0 mm in Figure 28A, and the decentration is set to zero.

[Figure 28E] Figure 28E is a diagram in which the aperture diameter is set to 4.0 mm and the decentration is set to 0.3 mm in Figure 28A.

[Figure 28F] Figure 28F is a diagram in which the aperture diameter is set to 4.0 mm and the decentration is set to 0.5 mm in Figure 28A.

[Figure 29A] Figure 29A is a plot showing the MTF value (vertical axis) indicating contrast with respect to the tilt (horizontal axis) (object distance set to 3.0 m, aperture diameter (pupil size) 3.0 mm, and no decentration).

[Figure 29B] Figure 29B is a diagram in which the decentration is set to 0.3 mm in Figure 29A.

[Figure 29C] Figure 29C is a diagram in which the decentration is set to 0.5 mm in Figure 29A.

[Figure 29D] Figure 29D is a diagram in which the aperture diameter is set to 4.0 mm in Figure 29A, and the decentration is set to 0 mm.

[Figure 29E] Figure 29E is a diagram in which the aperture diameter is set to 4.0 mm and the decentration is set to 0.3 mm in Figure 29A.

[Figure 29F] Figure 29F is a diagram in which the aperture diameter is set to 4.0 mm and the decentration is set to 0.5 mm in Figure 29A.

[Figure 30A] Figure 30A is a plot showing the MTF value (vertical axis) indicating contrast with respect to the tilt (horizontal axis) (object distance set to 4.0 m, the aperture diameter (pupil size) 3.0 mm, and no decentration).

[Figure 30B] Figure 30B is a diagram in which the decentration is set to 0.3 mm in Figure 30A.

[Figure 30C] Figure 30C is a diagram in which the decentration is set to 0.5 mm in Figure 30A.

[Figure 30D] Figure 30D is a diagram in which the aperture diameter is set to 4.0 mm in Figure 30A, and the decentration is set to zero.

[Figure 30E] Figure 30E is a diagram in which the aperture diameter is set to 4.0 mm and the decentration is set to 0.3 mm in Figure 30A.

[Figure 30F] Figure 30F is a diagram in which the aperture diameter is set to 4.0 mm and the decentration is set to 0.5 mm in Figure 30A.

[Figure 31A] Figure 31A is a schematic plan view showing an intraocular lens of Embodiment 5A of the present invention.

[Figure 31B] Figure 31B is a schematic plan view showing an intraocular lens of Embodiment 5B of the present invention.

[Figure 32A] Figure 32A is a power profile in which a position seen in a radial direction from a lens center O is set as a horizontal axis (unit: mm), and power is set as a vertical axis (unit: D (diopter)) in the intraocular lens of Embodiment 5A of the present invention.

[Figure 32B] Figure 32B is a power profile in which a position seen in a radial direction from a lens center O is set as a horizontal axis (unit: mm), and power is set as a vertical axis (unit: D (diopter)) in the intraocular lens of Embodiment 5B of the present invention.

[Figure 33A] Figure 33A is a diagram showing a schematic plan view (upper side) in the case of including an outer transition region in the intraocular lens of Embodiment 5A of the present invention, and a power profile (lower side) in which the position seen in the radial direction from the lens center O is set as a horizontal axis (unit: mm), and the power is set as a vertical axis (unit: D (diopter)).

[Figure 33B] Figure 33B is a diagram showing a schematic plan view (upper side) in the case of including an inner transition region in addition to an outer transition region in the intraocular lens of Embodiment 5B of the present invention, and a power profile (lower side) in which the position seen in the radial direction from the lens center O is set as a horizontal axis (unit: mm), and the power is set as a vertical axis (unit: D (diopter)).

[Figure 34] Figure 34 is a power profile in which the position seen in the radial direction from the lens center O is set as a horizontal axis (unit: mm), and the power is set as a vertical axis (unit: D (diopter)) in the intraocular lens of Embodiment 5A of the present invention, which is a diagram explaining a ratio of a range in the radial direction of a constant power addition region with respect to a range in the radial direction of an inner first region.

## Description of Embodiments

### [Definitions, etc.]

Note that for configurations that are not described below, known configurations may be adopted as appropriate. In particular, the contents disclosed in the literature (WO2009/153873) disclosed by the present inventors (especially the support portion among them) may be applied to the present embodiments.

Further, in the present specification, the term "to" indicates a value larger than or equal to a given value and less than or equal to a given value.

Further, a lens body of an intraocular lens used in the present specification has two surfaces facing each other. A surface of the lens body on a side coming into contact with a posterior capsule when the intraocular lens is inserted into a crystalline lens capsule can be referred to as a posterior surface, a surface on a retina side, or a surface on the retina side in an optical axis direction, but the "posterior surface" will be used primarily in the present specification. The other surface can be referred to as an anterior surface, a surface on a cornea side, a surface on the cornea side in the optical axis direction, but the "anterior surface" will be used primarily in the present specification. Further, the optical axis direction is also a lens thickness direction, and a direction toward the anterior surface from the posterior surface or an opposite direction thereto. The optical axis direction is a z-axis direction.

A lens center O refers to a geometric center or an optical center of the intraocular lens. In the present specification, the case in which the geometric center and the optical center coincide with each other is illustrated. Refractive power at the lens center O is referred to as base power. This base power refers to the refractive power required for so-called distance vision in conventional intraocular lenses.

### [Common Embodiment]

Hereinafter, embodiments of the present invention will be described in detail with reference to the drawings. First, contents and an inventive concept that are common to Embodiment 1 and subsequent embodiments will be described as a common embodiment.

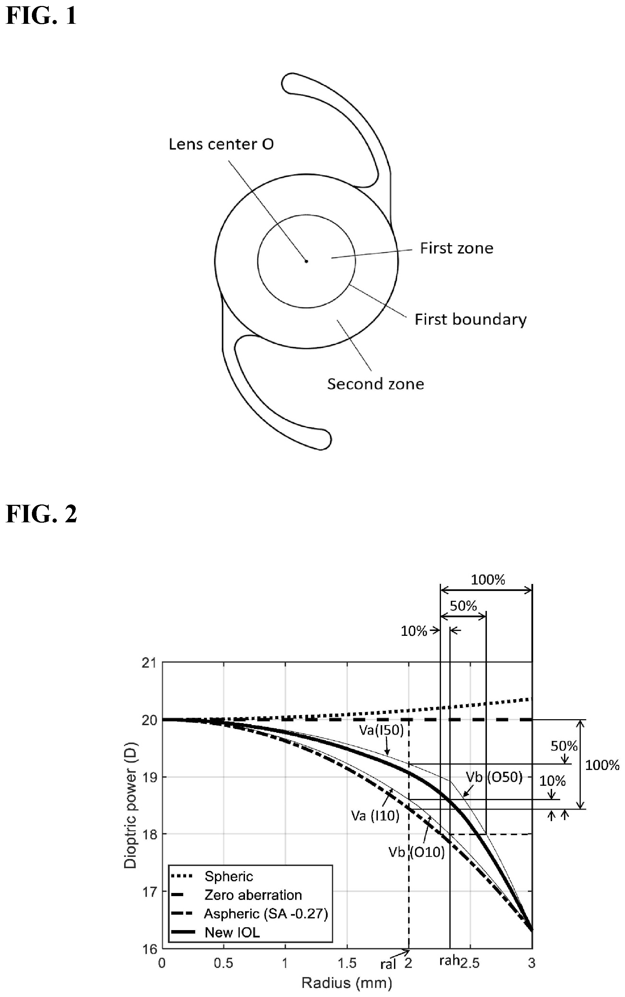

Figure 1 is a schematic plan view showing an intraocular lens of the common embodiment.

As shown in Figure 1, the intraocular lens according to the common embodiment (and the other aspects described in the present specification) includes a lens body having a lens function, and a support portion that supports the lens body in a crystallin lens capsule, as in the conventional intraocular lenses.

A material of the intraocular lens is not limited, and may be made of at least any one of silicone, hydrophobic acrylic resin, hydrophilic acrylic resin, hydrogel, PMMA, PMMA copolymer, and HEMA (hydroxyethyl methacrylate) (for example, collamer (registered trademark)) copolymer containing collagen.

As shown in Figure 1, in the common embodiment (and the other aspects described in the present specification), the case in which the entire lens body is an optical portion having a lens function is illustrated. The "lens function" mentioned here refers to a function of refracting incident light beams onto a retina.

In this specification, the entire lens body is exemplified as being composed of a first zone (zone 1) and a second zone (zone 2) described later. The other aspects described in this specification include a middle zone surrounding the first zone, between the first zone and the second zone, or includes an additional region surrounding the second zone, outside of the second zone in the radial direction.

In the common embodiment, the intraocular lens is defined by refractive power (power, diopter) with respect to a distance when being away from the lens center O in the radial direction. The "direction away from the lens center O in the radial direction" is set as "outside" in the present specification.

First, the aspect of the intraocular lens according to the common embodiment (and the other aspects described in the present specification) includes at least two zones for vision correction that are concentric with the lens center O to which a predetermined base power is set and are adjacent to each other. The zones for vision correction are set as, in order from the first zone including the lens center O outward in the radial direction, the first zone, and the second zone surrounding the first zone. Then, a position of a first boundary (boundary 1) between the first zone and the second zone when seen in the radial direction from the lens center O is set as r1.

As shown in Figure 1, in plan view, the first zone has a circular shape, and the second zone has a ring shape. In the other aspect described in the present specification, a middle zone has a small ring shape, and an additional region has a large ring shape. Note that they may have an elliptical shape and/or an elliptical ring shape instead of a circular shape

and/or a ring shape. In the case of an ellipse, the position r1 of the first boundary may adopt a major axis or a minor axis.

Figure 2 is a power profile in which a position when seen in the radial direction from the lens center O is set as a horizontal axis (unit: mm) and a power is set as a vertical axis (unit: D (diopter)). A distance from the lens center O is also referred to as a radius (radius).

The solid line represents a power profile of the intraocular lens according to the common embodiment (and the other aspects described in the present specification).

The dotted line represents a power profile of a virtual spherical lens (also referred to as "spherical IOL" in the present specification) having a base power (broken line) in the lens center O. Note that an intraocular lens in which the power is constant regardless of the value of the radius that is the horizontal axis is known (for example, SofPort (registered trademark) AO IOL). In the present specification, this type of intraocular lens is also referred to as a "zero aberration IOL". However, the zero aberration IOLs do not take the cornea into consideration, unlike aspherical reference power profiles W listed below. In other words, in a power profile of the zero aberration IOL, positive longitudinal spherical aberration and refractive power provided by the cornea are not taken into account.

An alternate long and short dash line represents a power profile of a virtual aspherical lens that has a base power in the lens center O, and completely cancels the positive longitudinal spherical aberration provided by the cornea. This power profile is also referred to as an aspherical reference power profile W.

The longitudinal spherical aberration is spherical aberration in a direction extending radially seen from the lens center O, that is, the radial direction (meridional). In this case, spherical aberration in a circumferential direction (sagittal) perpendicular to the radial direction is a transverse spherical aberration.

The cornea has positive refractive power. The further away from the center of the cornea, the larger the spherical aberration. In other words, it is the alternate long and short dash line showing the aspherical reference power profile W that plots the virtual aspherical lens that theoretically cancels the positive longitudinal spherical aberration provided by the cornea, completely.

Hereinafter, meanings of various lines are similar.

An aspherical optical design IOL including the aspherical reference power profile W is designed to correct or reduce all or part of the spherical aberration of the cornea. The degree to which the spherical aberration of the cornea is reduced varies for each company's IOL. The IOL of each company is designed to reduce the spherical aberration of the cornea by a specific amount (value). In the design of the aspherical IOL, the specific amount of the spherical aberration to be reduced by the aspherical IOL is determined by setting a corneal model including the same spherical aberration value as the specific amount of the spherical aberration to be reduced in advance, and selecting the optical parameter of the corneal model. During the optical design work, total spherical aberration of an optical system composed of the predetermined corneal model determined in advance and the designed aspherical IOL is zero (that is, there is no aspherical aberration).

The spherical aberration value of the predetermined corneal model is determined as follows. It is assumed that the spherical aberration value of a predetermined corneal model used in the optical design of an aspherical IOL is the same value as the average value of the spherical aberration for a group of eye patients wearing IOLs. Alternatively, the spherical aberration value of the predetermined corneal model is determined by setting the spherical aberration value to a spherical aberration value that partially decreases the cornea spherical aberration of the group of eye patients.

The aspherical reference power profile W is a power distribution of the aspherical IOL. This power distribution has power distribution characteristics that can completely or partially reduce the spherical aberration of an average cornea (statistical corneal optical parameter) of an aphakic patient group. In the present specification, a spherical aberration value of the predetermined corneal model is 0.27 μm. Figure 2 is also an example of a power distribution of an aspherical IOL that can completely reduce the spherical aberration of the corneal model with a spherical aberration value of 0.27 μm. 0.27 μm may be expressed as +0.27 μm. Furthermore, the present invention is not strictly limited to 0.27 μm and may be a value in a range of +0.24 to +0.30 μm, for example.

Hereinafter, the invention concept common to Embodiment 1 and the subsequent embodiments will be described.

Again, an object of the present invention is to provide the intraocular lens in which the change in image quality is robust with respect to changes in the above-described decentration, tilt, and pupil size (hereinafter, also collectively referred to as "respective changes") and the related technology thereof. The term "robust" mentioned here also means not easily influenced or insensitive.

The above-described aspherical IOL can correct or reduce all or part of the spherical aberration of a cornea and can meet prescription values. On the other hand, the change in image quality is sensitive to the above-described respective changes.

In the above-described spherical IOL, change in image quality is robust with respect to the above-described respective changes as compared with the aspherical IOL. On the other hand, in the absence of the above-described respective changes, the image quality of the spherical IOL is inferior to the image quality of the aspherical IOL. In particular, when the pupil size increases among the above-described respective changes, a difference in image quality is remark-

able.

In the above-described zero aberration IOL, change in image quality is robust with respect to the above-described respective changes as compared with the aspherical IOL. On the other hand, in the power profile of the zero aberration IOL, the positive longitudinal spherical aberration and the refractive power provided by the cornea are not taken into account. As a result, in the absence of the above-described respective changes, the image quality of the zero aberration IOL is a little better than that of the spherical IOL, but inferior to the image quality of the aspherical IOL.

In the present invention, reference will first be made to the aspherical reference power profile W of the aspherical IOL generally. In the aspherical reference power profile W, as the value of the horizontal axis that is in the radial direction from the lens center O increases, the value of the vertical axis that is the power decreases.

Here, the aspherical reference power profile W is divided into at least to zones. A side near the lens center O is the above-described first zone, and the outside thereof is the second zone. In the respective zones, the aspherical reference power profile W is modified.

In the first zone, a decrease rate of the vertical axis value is made more gradual than in the aspherical reference power profile W even when the horizontal axis value increases. In order to compensate this, in the second zone, the decrease rate of the vertical axis value is made more rapid than in the aspherical reference power profile W when the horizontal axis value increases.

As shown in various data shown below, in the intraocular lens that adopts the power profile obtained by modifying the aspherical reference power profile W with the concept like this, the change in the image quality is robust with respect to the above-described respective changes while the image quality remains as good as that of the aspherical IOL.

In other words, the concept of the present invention is to patch together two or more mutually different power profiles, rather than a single power profile such as the aspherical reference power profile W. The concept of the present invention is that, during the patching, in the first zone, the rate of decrease in the vertical axis value is made more gradual than in the aspherical reference power profile W when seen as a whole, while in the second zone, the rate of decrease in the vertical axis value is made more rapid than in the aspherical reference power profile W when seen as a whole.

Note that the concept of the present invention also has an advantage that the thickness of the intraocular lens can be reduced. For example, the central thicknesses of the spherical IOL, zero aberration IOL, aspherical IOL, and the IOL of the concept of the present invention (Figure 2 shown below) were measured under the following parameters. The parameters are also referred to as condition 1. The radius of curvature of the posterior surface is negative because the anterior surface is convex and the curving direction of the convex is positive.

| | |
|---|---|
| lens optical diameter | : 6 mm |
| refractive index at 35°C | : 1.520 |
| outermost edge thickness of lens body | : 0.18 mm |
| Radius of curvature of posterior surface | : -20.0 mm |

The result is as follows:

| | |
|---|---|
| Spherical IOL : | 0.674 mm |
| Zero aberration IOL : | 0.669 mm |
| Aspherical IOL : | 0.626 mm |
| IOL of concept of the present invention: | 0.632 mm |

In other words, the IOL of the concept of the present invention can reduce the thickness of the intraocular lens. This makes it easier to fold the intraocular lens and then restore it in the eye. In addition, the wound during the procedure can be reduced.

Embodiments 1 to 4 described hereinafter are specific aspects based on the above-described concept.

Embodiment 1 is an aspect in which a range in which a power profile V of an intraocular lens is included is expressed by a modification range of the aspherical reference power profile W in the first zone, and a modification range of the aspherical reference power profile W in the second zone based on the above-described concept.

Embodiment 2 is an aspect in which the above-described concept is expressed by an increase or decrease in values obtained by subtracting the vertical axis value of the aspherical reference power profile W from vertical axis values of power profiles V1 and V2.

Embodiment 3 is an aspect in which a middle zone is provided between the first zone and the second zone in Embodiment 1.

Embodiment 4 is an aspect in which the power profile in Embodiment 1 is converted as a total power profile in

which refractive power of a cornea is added.

Note that intraocular lenses according to Embodiments 1 to 4 described hereinafter are aspherical intraocular lenses. A surface of the intraocular lens that is subjected to lathe-turning (formed) into an aspherical surface to realize (create) the power profile taught by the contents (specification of the present application) laid-open by the present application may be applied to only an anterior surface, only a posterior surface, or the anterior surface and the posterior surface (both surfaces).

[Embodiment 1]

An intraocular lens according to Embodiment 1 is as follows. Embodiment 1 and the subsequent embodiments can be optionally combined with each other with respect to each of the embodiments (including the common embodiment). Also, contents described in Embodiment 1 can be applied to each of the other embodiments (including the common embodiment). "An intraocular lens including a power profile V that is included within a region of a set of power profiles obtained by respective combinations of power profiles Va and Vb, when an intersection point of

the power profile Va for a first zone created by adding values obtained by multiplying values obtained by subtracting vertical axis values in respective horizontal axis values of an aspherical reference power profile W from a base power by a predetermined ratio $\alpha$ ($\alpha$ is 10% or more and 50% or less) to the vertical axis values in the respective horizontal axis values of the aspherical reference power profile W, and

the power profile Vb for a second zone created by adding values obtained by multiplying values obtained by subtracting the horizontal axis values in the respective vertical axis values of the aspherical reference power profile W from a maximum value rmax in a horizontal axis of the aspherical reference power profile W by a predetermined ratio $\beta$ ($\beta$ is 10% or more and 50% or less) to the horizontal axis values in the respective vertical axis values of the aspherical reference power profile W

is a position ra in a horizontal axis".

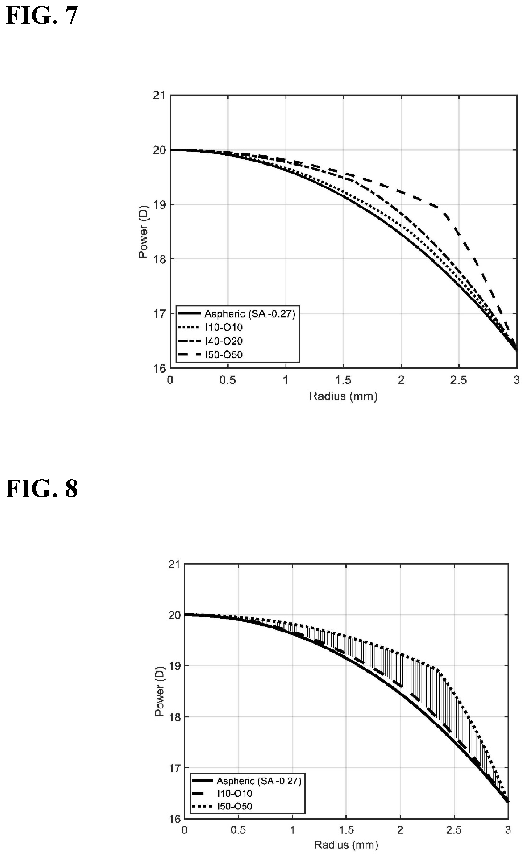

"A region formed by the power profile when the intersection point of the power profile Va for the first zone, and the power profile Vb for the second zone is the position ra in the horizontal axis (hereinafter, also referred to as a "set region") " refers to a hatched portion in Figure 8. The power profile Va and the power profile Vb intersect each other. The power profile Va is adopted as for the first zone in a negative direction of the horizontal axis from this intersection point (horizontal axis position ra). The power profile Vb is adopted as for the second zone in a positive direction of the horizontal axis from this intersection point (horizontal axis position ra). The set region of the power profiles obtained by the respective combinations in which $\alpha$ is 10% or more and 50% or less, and $\beta$ is 10% or more and 50% or less is the hatched portion in Figure 8.

The maximum value rmax of the horizontal axis of the aspherical reference power profile W means an outermost edge of a lens body, that is, an optical portion having a lens function.

The contents concerning the above-described "multiplying by predetermined ratios $\alpha$, $\beta$" may be expressed as follows.

"An intraocular lens including a power profile V that is included within a region of a set of power profiles obtained by respective combinations of power profiles Va and Vb, when an intersection point of

the power profile Va for a first zone obtained by reducing an aspherical reference power profile W by a constant scale factor $\alpha$ ($\alpha$ is 10% or more and 50% or less) from a negative direction to a positive direction of a vertical axis (while leaving the portion corresponding to the vertical axis value (base power) when the horizontal axis value being zero unchanged), and

the power profile Vb for a second zone obtained by reducing the aspherical reference power profile W by a constant scale factor $\beta$ ($\beta$ is 10% or more and 50% or less) from a negative direction to a positive direction of a horizontal axis (while leaving the portion corresponding to the horizontal axis value rmax unchanged)

is a position ra in a horizontal axis."

For example, r1 is a value within a range of 1.5 mm or more and 2.3 mm or less.

For example, in respective horizontal axis values of the power profile V, among values obtained by subtracting the vertical axis values of the aspherical reference power profile W from the vertical axis values of the power profile V, a position r1 has a positive maximum value. The position r1 is also a spot where mutually different power profiles are patched together.

For example, the maximum value rmax of the horizontal axis of the power profile V is a value within a range of 2.5 mm or more and 3.5 mm or less. Hereinafter, unless otherwise specified, the case where rmax is 3.0 mm will be exemplified.

The intraocular lens according to the present embodiment may include one or more additional regions toward the radial direction from the second zone. An additional region surrounds the second zone. The additional region preferably includes a function of refracting incident light beams onto a retina, and preferably exhibits a function included by an optical portion.

In respective horizontal axis values of a power profile Vadd in at least one region of the additional regions, a value obtained by subtracting the vertical axis value of the aspherical reference power profile W from vertical axis values of the power profile Vadd may be less than ±0.30 D (preferably, less than ±0.15 D, same below). In other words, at least one region (preferably an additional region located on an outermost side, or only one additional region) of the additional regions located outside of the second zone may be consistent or inconsistent with the aspherical reference power profile W with a deviation of less than ±0.30 D on the vertical axis.

As described above, the aspherical IOL has good image quality in conditions without the above-described respective changes. The effect that the change in image quality against the above-described respective changes is robust is substantially achieved in the above-described first zone and second zone. As a result, even if the additional region is provided outside the second zone, it is preferable to adopt a power profile that resembles the aspherical reference power profile W as closely as possible. Deviation from the plot of the aspherical reference power means that longitudinal spherical aberration occurs, which in turn leads to poor vision correction. In order to avoid this, it is preferable to adopt a power profile that resembles the aspherical reference power profile W as closely as possible.

The power profile V may be expressed as a polynomial expression. For example, respective power profiles of the above-described first zone, second zone, a middle zone shown below, and the above-described additional region can be expressed by the following polynomial expression.

$$P(r) = a_n r^n + a_{n-1} r^{n-1} + a_{n-2} r^{n-2} + a_{n-3} r^{n-3} + \quad \ldots \quad + a_3 r^3 + a_2 r^2 + a_1 r^1 + a_0$$

$$\cdots \text{(Expression 1)}$$

P(r): power with respect to lens radius position

a: coefficient
r: lens radius position
n: polynomial degree

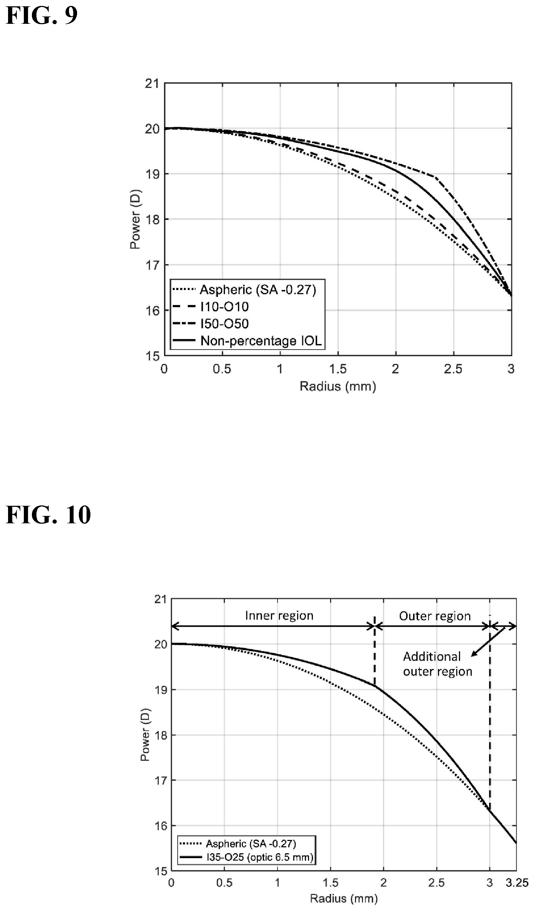

The following polynomial expressions are polynomial expressions in the respective zones and region when the position r1 is 1.92 mm, the horizontal axis value of 3.00 mm is a position of a boundary between the second zone and the additional region (only one), and rmax is 3.25 mm (see Figure 10). Note that I35 in $P_{I35}$ below indicates that the above-described predetermined ratio $\alpha$ in the power profile Va is 35% (when $\alpha$ is 10%, it is described as Va (I10) as shown in Figure 2, for example), and O25 in $P_{O25}$ indicates that the above-described predetermined ratio $\beta$ in the power profile Vb is 25% (when $\beta$ is 10%, it is described as Vb (O10) as shown in Figure 2, for example).

The first zone:

$$P_{I35}(r)=-2.87802590e-03r^4-4.84408818e-04r^3-2.39306469e-01r^2-1.30725682e-04r+20.000009$$

The second zone:

$$P_{O25}(r)=-4.38903190e-04r^4-7.72003537e-02r^3-3.13958521e-01r^2+4.43293306e-01r+19.934990$$

The additional region:

$$P_{add}(r)=1.75786855r^4-2.21096892e+01r^3+1.03671760e+02r^2-2.17611350e+02r+190.681131$$

In the present embodiment, when seen in Figure 2, a horizontal axis value of an intersection point of $P_{I50}$ and $P_{O50}$ is rah, and a horizontal axis value of an intersection point of $P_{O10}$ and $P_{I10}$ is ral. In other words, when seen in Figure 2, the power profile V is included within a region (hatched portion in Figure 8) surrounded by $P_{I50}$, rah, $P_{O50}$, $P_{O10}$, ral, and $P_{I10}$ in a clockwise order.

However, the present invention is not limited to the power profile V being included within the above-described region (hatched portion in Figure 8). For example, even if the vertical axis value of the power profile V is slightly lower than the aspherical reference power profile W in the horizontal axis value of the power profile V that is zero or a value close to

rmax, the effect on the optical performance as an intraocular lens (IOL) is small, and in addition, the change in image quality remains robust against the above-described respective changes. The following Embodiment 2 is a provision that strongly reflects the concept of the above-described invention, taking the above into consideration.

[Embodiment 2]

An intraocular lens according to Embodiment 2 is as follows.
"An intraocular lens, in which

in a first zone, an average value of vertical axis values of a power profile V1 for the first zone is larger than an average value of vertical axis values of an aspherical reference power profile W,
in a second zone, an average value of vertical axis values of a power profile V2 for the second zone is smaller than an average value of the vertical axis values of the aspherical reference power profile W,
in the power profile V1 for the first zone, with increase in horizontal axis values, values obtained by subtracting the vertical axis values of the aspherical reference power profile W from the vertical axis values of the power profile V1, in the respective horizontal axis values continuously increases,
in the power profile V2 for the second zone, with increase in horizontal axis values, values obtained by subtracting the vertical axis values of the aspherical reference power profile W from the vertical axis values of the power profile V2, in respective horizontal axis values continuously decreases,
in a power profile V, a position r1 has a positive maximum value among values obtained by subtracting the vertical axis values of the aspherical reference power profile W from the vertical axis values of the power profile V2, and
a value obtained by subtracting the vertical axis value of the aspherical reference power profile W from a vertical axis value at a maximum value rmax of a horizontal axis of the power profile V is less than 0.25 D."

For example, an absolute value of an average value of a slope of a tangent line of the power profile V2 in the second zone is equal to or larger than three times an absolute value of an average value of a slope of a tangent line of the power profile V1 in the first zone. In other words, in the power profile V of the intraocular lens according to the present embodiment, the power profiles having the absolute values of the average values of the slopes of the tangent lines that are different from each other by this amount are patched together at the position r1.

The meaning of "in a first zone, an average value of vertical axis values of a power profile V1 for the first zone is larger than an average value of vertical axis values of an aspherical reference power profile W" is as follows. It means that the average power in the first zone of the intraocular lens according to the present embodiment is larger than the average power of the aspherical reference power profile W in the first zone. Similarly, "in a second zone, an average value of vertical axis values of a power profile V2 for the second zone is smaller than an average value of the vertical axis values of the aspherical reference power profile W" means that the average power in the second zone of the intraocular lens according to the present embodiment is smaller than the average power of the aspherical reference power profile W in the second zone.

Note that "value obtained by subtracting in the first zone continuously increases" and "value obtained by subtracting in the second zone continuously decreases" are preferable examples, and can be excluded from the provisions. Even if there is only a part that does not satisfy these requirements in the first zone or the second zone, the influence on the effect of the present invention is minor.

Furthermore, together with the provisions that

"in a first zone, an average value of vertical axis values of a power profile V1 for the first zone is larger than an average value of vertical axis values of an aspherical reference power profile W,
in a second zone, an average value of vertical axis values of a power profile V2 for the second zone is smaller than an average value of the vertical axis values of the aspherical reference power profile W," among the above-described configurations of the intraocular lens according to Embodiment 2,
provisions that
"an absolute value of an average value of a slope of a tangent line of the power profile V2 in the second zone is larger than an absolute value of an average value of a slope of a tangent line of the power profile V1 in the first zone",
"in a power profile V, a position r1 has a positive maximum value among values obtained by subtracting the vertical axis values of the aspherical reference power profile W from the vertical axis values of the power profile V2", and
"r1 is a value within a range of 1.5 mm or more and 2.3 mm or less" are adopted as the provisions of the intraocular lens according to Embodiment 2.

For example, r1 is a value in a range of 1.5 mm or more and 2.3 mm or less. Further, the maximum value rmax in the horizontal axis of the power profile V is a value in a range of 2.5 mm or more and 3.5 mm or less.

The following provision adopted in Embodiment 1 may be adopted in the present embodiment.
"including a power profile V that is included within a region of a set of power profiles obtained by respective combinations of power profiles Va and Vb, when an intersection point of

the power profile Va for a first zone created by adding values obtained by multiplying values obtained by subtracting vertical axis values in respective horizontal axis values of an aspherical reference power profile W from a base power by a predetermined ratio $\alpha$ ($\alpha$ is 10% or more and 50% or less) to the vertical axis values in the respective horizontal axis values of the aspherical reference power profile W, and
the power profile Vb for a second zone created by adding values obtained by multiplying values obtained by subtracting the horizontal axis values in the respective vertical axis values of the aspherical reference power profile W from a maximum value rmax in a horizontal axis of the aspherical reference power profile W by a predetermined ratio $\beta$ ($\beta$ is 10% or more and 50% or less) to the horizontal axis values in the respective vertical axis values of the aspherical reference power profile W

is a position ra in the horizontal axis."
One or more additional regions are included toward outside in the radial direction from the second zone, the additional regions surround the second zone, and in the respective horizontal axis values of the power profile Vadd in at least one region of the additional regions, values obtained by subtracting the vertical axis values of the aspherical reference power profile W from the vertical axis values of the power profile Vadd may be less than $\pm 0.30$ D. The additional region may include the function of refracting incident light beams onto a retina.
The power profile V may be expressed by the above-described polynomial expression.

[Embodiment 3]

An intraocular lens according to Embodiment 3 is as follows.
"An intraocular lens including a power profile V that is included within a region of a set of power profiles obtained by respective combinations of power profiles Va, Vmid and Vb, composed of

the power profile Va for a first zone, created by adding values obtained by multiplying values obtained by subtracting vertical axis values in respective horizontal axis values of an aspherical reference power profile W from a base power by a predetermined ratio $\alpha$ ($\alpha$ is 10% or more and 50% or less) to the vertical axis values in the respective horizontal axis values of the aspherical reference power profile W,
the power profile Vb for a second zone, created by adding values obtained by multiplying values obtained by subtracting the horizontal axis values in the respective vertical axis values of the aspherical reference power profile W from a maximum value rmax in a horizontal axis of the aspherical reference power profile W by a predetermined ratio $\beta$ ($\beta$ is 10% or more and 50% or less) to the horizontal axis values in the respective vertical axis values of the aspherical reference power profile W, and
the power profile Vmid for a middle zone in which an average power is smaller than an average power in the first zone, and is larger than an average power in the second zone."

The middle zone in the present embodiment includes a role of connecting the first zone and the second zone. The middle zone may serve as a transition region that smoothly connects the first zone and the second zone in the vertical axis value. In other words, in the present embodiment, when mutually different power profiles are patched together, another power profile is inserted between the power profiles, and thereafter the three power profiles are pathed together, instead of directly patching together the mutually different power profiles.
For example, an absolute value of an average value of a slope of a tangent line of the power profile Vmid in the middle zone is larger than an absolute value of an average value of a slope of a tangent line of a power profile V1 in the first zone, and is smaller than an absolute value of an average value of a slope of a tangent line of a power profile V2 in the second zone.
For example, in respective horizontal axis values of the power profile Vmid in the middle zone, an average value of values obtained by subtracting vertical axis values of the aspherical reference power profile W from vertical axis values of the power profile Vmid is

larger than an average value of values obtained by subtracting vertical axis values of the aspherical reference power profile W from vertical axis values of the power profile V1 in respective horizontal axis values of the power profile V1 in the first zone, and
larger than an average value of values obtained by subtracting vertical axis values of the aspherical reference power profile W from vertical axis values of the power profile V2 in respective horizontal axis values of the power profile V2 in

the second zone.

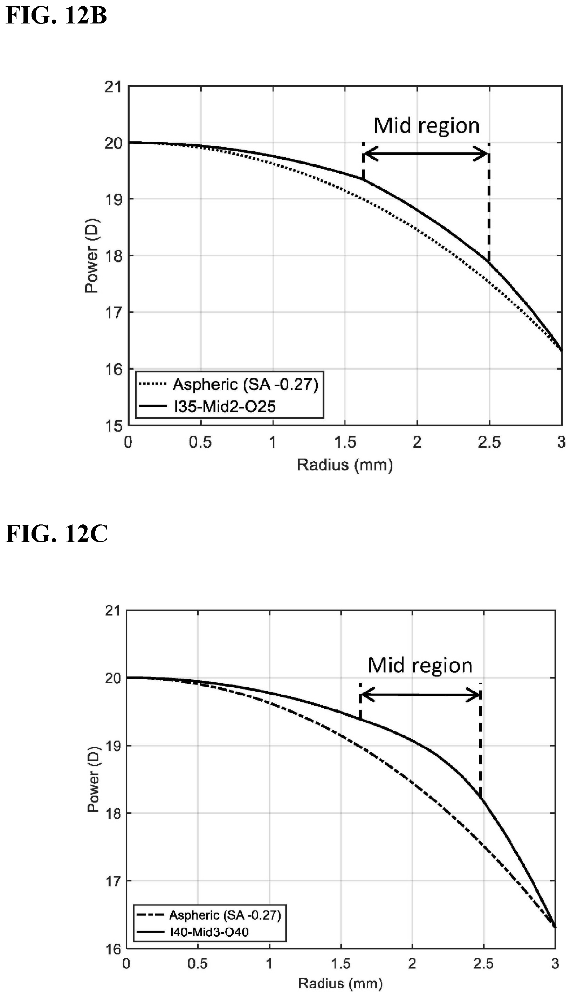

For example, in the middle zone, the horizontal axis values are included within a range of 1.3 mm or more and 2.5 mm or less.
For example, the maximum value rmax of the horizontal axis of the power profile V is a value within a range of 2.5 mm or more and 3.5 mm or less.
For example, the absolute value of the average value of the slope of the tangent line of the power profile V2 in the second zone is equal to or more than three times the absolute value of the average value of the slope of the tangent line of the power profile V1 in the first zone.
For example, one or more additional regions are included toward outside in the radial direction from the second zone, the additional regions surround the second zone, and a value obtained by subtracting vertical axis values of the aspherical reference power profile W from the vertical axis values of the power profile Vadd in the respective horizontal axis values of the power profile Vadd in at least one region of the additional regions is less than ±0.30 D. The additional region includes a function of refracting incident light beams onto a retina.
For example, the power profile V is expressed by a polynomial expression.
The following polynomial expressions are polynomial expressions in the respective zones and region when a boundary position between the first zone and the middle zone is 1.625 mm, a boundary position between the middle zone and the second zone is 2.485 mm, and rmax is 3.000 mm (See Figure 12B).
The first zone:

$$P_{I35}(r)=-2.87802590e\text{-}03r^4-4.84408818e\text{-}04r^3-2.39306469e\text{-}01r^2-1.30725682e\text{-}04r+20.000009$$

The middle zone:

$$P_{mid}(r)=-3.75928693e\text{-}03r^4-3.53365485e\text{-}03r^3-3.65323646e\text{-}01r^2+1.15043830e\text{-}03r+20.347617$$

The second zone:

$$P_{O25}(r)=-4.38903190e\text{-}04r^4-7.72003537e\text{-}02r^3-3.13958521e\text{-}01r^2+4.43293306e\text{-}01r+19.934990$$

[Embodiment 4]

An intraocular lens according to Embodiment 4 is as follows.
"An intraocular lens including a total power profile TV included within a region of a set of total power profiles obtained by respective combinations of total power profiles TVa and TVb when an intersection point of

the total power profile TVa for a first zone created by adding values obtained by multiplying values obtained by subtracting vertical axis values in respective horizontal axis values of an aspherical reference total power profile TW in which the refractive power of a cornea is added from a base power by a predetermined ratio $\alpha$ ($\alpha$ is 10% or more and 50% or less) to the vertical axis values in the respective horizontal axis values of the aspherical reference total power profile TW, and
the total power profile TVb for a second zone created by adding values obtained by multiplying values obtained by subtracting the horizontal axis values in the respective vertical axis values of the aspherical reference total power profile TW in which the refractive power of the cornea is added from a maximum value rmax of a horizontal axis of the aspherical reference total power profile TW by a predetermined ratio $\beta$ ($\beta$ is 10% or more and 50% or less) to the horizontal axis values in the respective vertical axis values of the aspherical reference total power profile TW

is a position ra in a horizontal axis,
when a position of a first boundary between the first zone and the second zone when seen in a radial direction from a lens center O is r1, in a total power profile in which a position when seen in the radial direction from the lens center O is set as a horizontal axis (unit: mm), and a total power T at a time of adding up refractive power of a cornea and power of the intraocular lens is set as a vertical axis (unit: D (diopter))."

In a total power profile TV1 for the first zone, a vertical axis value may continuously increase, with increase in a horizontal axis value, and
in a total power profile TV2 for the second zone, a vertical axis value may continuously decrease with increase in a

horizontal axis value.

An absolute value of an average value of a slope of a tangent line of the total power profile TV1 in a vicinity of an origin in the first zone may be smaller than an absolute value of an average value of a slope of a tangent line of the total power profile TV1 in a vicinity of a middle position in the first zone,

the absolute value of the average value of the slope of the tangent line of the total power profile TV1 in the vicinity of the middle position in the first zone may be larger than an absolute value of an average value of a slope of a tangent line of the total power profile TV1 in a vicinity of a position r1 in the first zone, and
the absolute value of the average value of the slope of the tangent line of the total power profile TV1 in the vicinity of the position r1 in the first zone may be smaller than an absolute value of an average value of a slope of a tangent line of the total power profile TV2 in a vicinity of a middle position in the second zone.

The "vicinity" mentioned here refers to within $\pm$0.20 mm (or 0.10 mm) from the middle position in the case of the middle position, for example.

For example, in the total power profile TV, a value obtained by subtracting a vertical axis value in r=0 from a vertical axis value in the position r1 is a positive maximum value, and
r1 is a value within a range of 1.5 mm or more and 2.3 mm or less.

For example, a value obtained by subtracting the vertical axis value in r=0 from a vertical axis value in the maximum value rmax of the horizontal axis of the total power profile TV is less than $\pm$0.30 D.

For example, the maximum value rmax of the horizontal axis of the total power profile TV is a value within a range of 2.5 mm or more and 3.5 mm or less.

For example, one or more additional regions are included toward outside in the radial direction from the second zone, and the additional regions surround the second zone, and the additional regions include a function of refracting incident light beams onto a retina.

[Others]

The intraocular lens of the present embodiment is not limited to the aforementioned embodiments, and also includes modes in which various modifications and improvements are added within the scope in which the special effects obtained by the components of the invention and the combinations thereof can be derived.

An area ratio of the first zone and the second zone in plan view may be set to 25:75 to 75:25.

It is preferable that the power continuously decrease in the first zone.

It is preferable that the power continuously decrease in the second zone.

It is preferable that the total power T at the time of the refractive power of a cornea and the power of the intraocular lens being added up continuously increase between the lens center O and the position r1 of the first boundary.

Rather than a profile obtained by directly patching together mutually different power profiles, a profile that resembles the patched profile may be adopted. A polynomial expression showing the power profile like this is as follows.

$$P_{Np}(r) = -8.31778546e\text{-}02r^8 + 9.97022762e\text{-}01r^7 - 4.79532863e{+}00r^6 + 1.18428403e{+}01r^5 - 1.60518818e{+}01r^4 + 1.18152492e{+}01r^3 - 4.57931927e{+}00r^2 + 6.59257903e\text{-}01r + 19.976408$$

Furthermore, although in each of the embodiments, the intraocular lens is illustrated, the technical idea (concept) of the present invention is also applicable to a design method of an intraocular lens. The technical idea (concept) of the present invention is also applicable to a manufacturing method of an intraocular lens for manufacturing the intraocular lens designed by the design method of an intraocular lens by at least any one of lathe-turning, molding, and 3D printing.

[Specific Examples]

The intraocular lenses of the present embodiments are not limited to the aforementioned embodiments, and also include modes in which various modifications and improvements are added in a scope in which specific effects obtained by the components of the invention and combinations thereof can be derived.

Hereinafter, specific examples of the power profile will be described. In the following specific examples, the parameters of the above-described condition 1 are adopted.

Figure 2 is the power profile showing refractive power (vertical axis) provided by the optical portion of an intraocular lens, with respect to the distance (horizontal axis) from the lens center O in the embodiments of the present invention.

In Figure 2, a power profile of the spherical IOL is shown by a dotted line, a power profile of the zero aberration IOL

is shown by a broken line, the power profile (aspherical reference power profile W) of the aspherical IOL is shown by an alternate long and short dash line, and the IOL of the concept of the present invention is shown by a solid line. In Figure 2, the power profile V is included within a region (hatched portion) surrounded by $P_{I50}$, $P_{O50}$, $P_{O10}$, and $P_{I10}$ in the clockwise order.

Figure 3 is a diagram showing a power profile showing refractive power (vertical axis) provided by the optical portion of the intraocular lens with respect to the distance (horizontal axis) from the lens center O, which is the power profile Va obtained by multiplying the value obtained by subtracting the vertical axis values in the respective horizontal axis values of the aspherical reference power profile W by the predetermined ratio $\alpha$ ($\alpha$ is 10%, that is, I10, 35%, that is, I35, 50%, that is, I50 among the values of 10% or more and 50% or less).

Figure 4 is a diagram showing a power profile showing refractive power (vertical axis) provided by the optical portion of the intraocular lens with respect to the distance (horizontal axis) from the lens center O, which is the power profile Vb created by adding the value obtained by multiplying the value obtained by subtracting the horizontal axis values in the respective vertical axis values of the aspherical reference power profile W from the maximum value rmax of the horizontal axis of the aspherical reference power profile W by the predetermined ratio $\beta$ ($\beta$ is 10%, that is, O10, 25%, that is, O25, 50%, that is, O50 among the values of 10% or more and 50% or less) to the horizontal axis values in the respective vertical axis values of the aspherical reference power profile W.

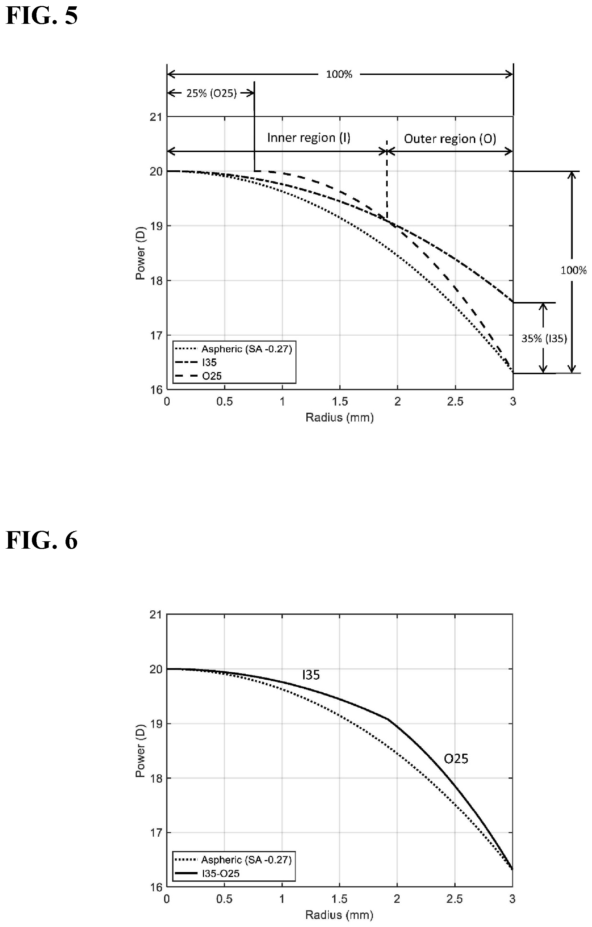

Figure 5 is an explanatory diagram at a time of I35 in Figure 3 and O25 in Figure 4 being superimposed on each other.

Figure 6 is a diagram showing the power profile V created by patching together the power profile Va (I35) for the first zone and the power profile Vb (O25) for the second zone in Figure 5.

Hereinafter, the power profile V is described as "I35-O25". Hereinafter, the power profile V will be described in a similar manner.

In both Figure 5 and Figure 6, the aspherical reference power profile W is shown as a dotted line. In the subsequent drawings, the aspherical reference power profile W is shown as appropriate.

Figure 7 is a diagram showing I10-O10, I40-O20, and I50-050.

In Figure 7, the aspherical reference power profile W is shown as a solid line.

Figure 8 is a diagram showing the region (hatched portion) composed of the power profiles when the intersection point of the power profile Va for the first zone ($\alpha$ is 10% or more and 50% or less), and the power profile Vb for the second zone ($\beta$ is 10% or more and 50% or less) is the position ra in the horizontal axis.

Figure 9 is a diagram showing the power profile V (solid line) when what is created by directly patching together mutually different power profiles is not adopted, but the profile that resembles what is created by patching together the mutually different power profiles is adopted. The aspherical reference power profile W (dotted line), I10-O10 (broken line), and I50-O50 (alternate long and short dash line) are also written.

Figure 10 is a diagram showing the power profile V (solid line, I35-O25, optical portion diameter 6.50 mm) in the case of being provided with an additional region.

The following specific examples are mainly related to Embodiment 2.

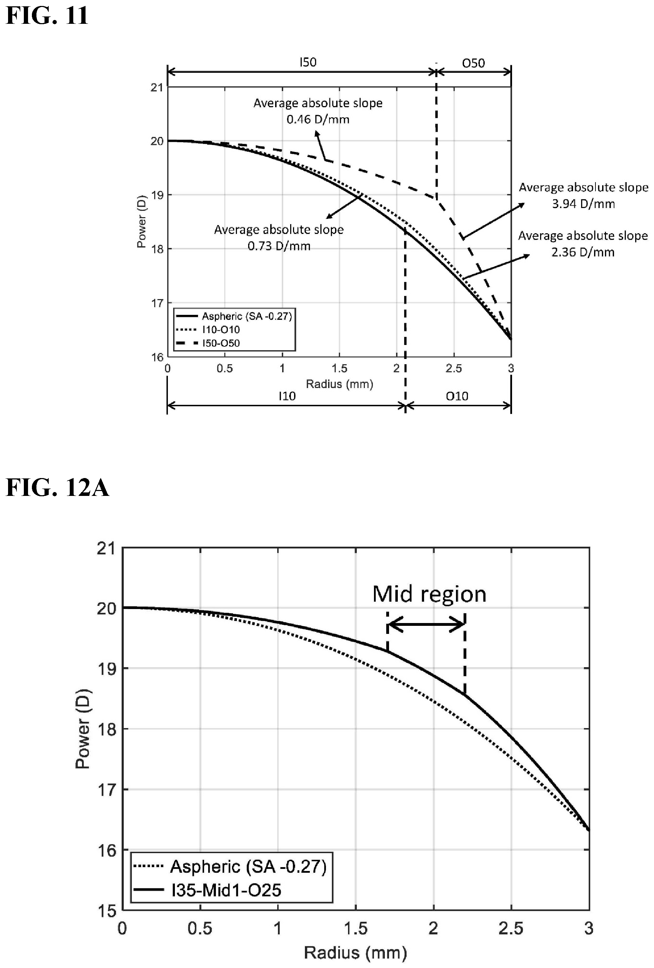

Figure 11 is a diagram showing I10-O10 and 150-050, which is a diagram in which an absolute value of an average value of a slope of a tangent line of the power profile V1 in the first zone and an absolute value of an average value of a slope of a tangent line of the power profile V2 in the second zone are both written with respect to each of the power profiles V.

In both of I10-O10 and 150-050 in Figure 11,

in the power profile V1 for the first zone, with increase in the horizontal axis value, the value obtained by subtracting the vertical axis value of the aspherical reference power profile W from the vertical axis value of the power profile V1 continuously increases in each of the horizontal axis value,

in the power profile V2 for the second zone, with increase in the horizontal axis value, the value obtained by subtracting the vertical axis value of the aspherical reference power profile W from the vertical axis value of the power profile V2 continuously decreases in each of the horizontal axis values,

in the power profile V, the position r1 has a positive maximum value among values obtained by subtracting the vertical axis values of the aspherical reference power profile W from the vertical axis values of the power profile V2, and the value obtained by subtracting the vertical axis value of the aspherical reference power profile W from the vertical axis value at the maximum value rmax of the horizontal axis of the power profile V is less than 0.25 D.

Furthermore,
the absolute value of the average value of the slope of the tangent line of the power profile V2 in the second zone is equal to or larger than three times the absolute value of the average value of the slope of the tangent line of the power profile V1 in the first zone.

In each of the horizontal axis values of the power profile Vadd in the additional region, the value obtained by

subtracting the vertical axis value of the aspherical reference power profile W from the vertical axis value of the power profile Vadd is less than ±0.30 D.

As a numerical range of the absolute value of the average value of the slope of the tangent line of the power profile V1 in the first zone, for example, 0.73 D/mm is cited as the upper limit, and 0.46 D/mm is cited as the lower limit.

As a numerical range of the absolute value of the average value of the slope of the tangent line of the power profile V2 in the second zone, for example, 3.94 D/mm is cited as the upper limit, and 2.36 D/mm is cited as the lower limit.

The following specific examples are mainly related to Embodiment 3.

Figure 12A is a diagram showing the power profile V (solid line, I35-O25) in the case of being provided with a middle zone.

Figure 12B is a diagram of the case where the middle zone in Figure 12A is widened.

Figure 12C is a diagram showing the power profile V (solid line, I40-O40) in the case of being provided with a middle zone.

The following specific examples are mainly related to Embodiment 4.

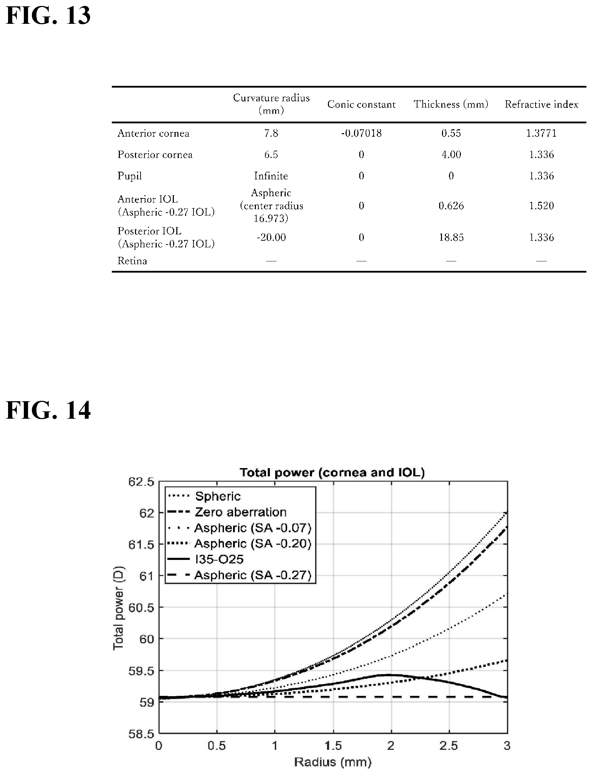

Figure 13 is a table showing conditions adopted in the total power profile according to the embodiment of the present invention.

Figure 14 is the total power profile TV showing a total power T (vertical axis) when the refractive power of the cornea and the power of the intraocular lens are added up, with respect to the distance (horizontal axis) from the lens center O.

As for the total power profile in Figure 14, the present example is designed by using optical design software ZEMAX (registered trademark) (made by ZEMAX Development Corporation of the United States) that is widely known, modeling the optical system of the cornea and IOL, and designing the aspherical anterior surface in the IOL.

In Figure 14, the power profile of the spherical IOL is written by a dotted line, the power profile of the zero aberration IOL by a broken line, and the IOL of the concept of the present invention by a solid line. Note that as for the total power profile of the aspherical IOL, a total power profile in the case of a spherical aberration value (described as a negative value in each of the drawings) of the corneal model being 0.07 μm is written by a wide dotted line, a total power profile in the case of the spherical aberration value being 0.20 μm is written by a narrow dotted line, and a total power profile in the case of the spherical aberration value being 0.27 μm is written by an alternate long and short dash line.

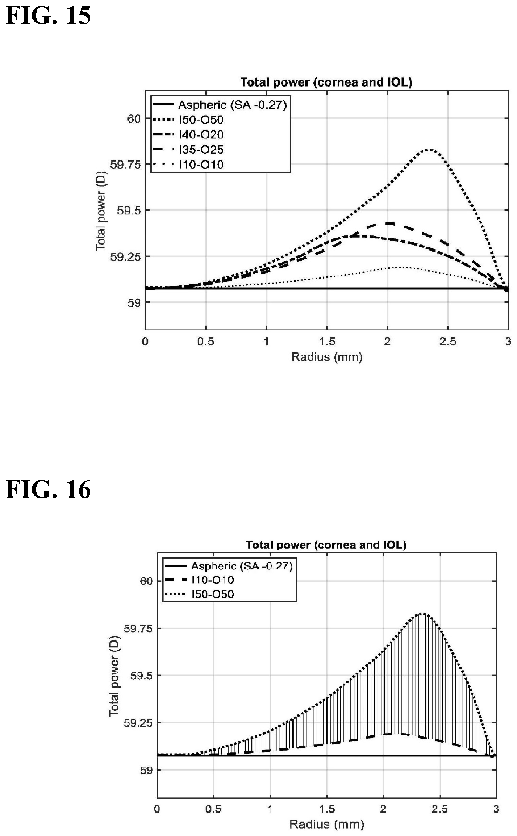

Figure 15 is a diagram showing a total power profile TV in the case where in the horizontal axis value rmax of 3.0 mm, the vertical axis value is matched with the vertical axis value at the time of the horizontal axis value being zero.

Figure 16 is a diagram showing a region (hatched portion) composed of total power profiles when the intersection point of the total power profile TVa for the first zone ($\alpha$ is 10% or more and 50% or less) and the total power profile TVb for the second zone ($\beta$ is 10% or more and 50% or less) is the position ra in the horizontal axis.

Hereinafter, it is shown that specific examples according to the present invention provides the effect that the change in image quality becomes robust against changes in the above-described decentration, tilt, and pupil size while the image quality remains good. In at least either of I40-O40 or 135-025 in the following specific example, the change in image quality is robust even when the above-described respective changes are present, while exhibiting the MTF value comparable to the aspherical IOL. The level of this robustness is comparable to that of a spherical IOL or a zero aberration IOL. In other words, the present invention makes it possible to combine the best points of a spherical IOL, zero aberration IOL, and aspherical IOL.

Beforehand, the MTF value in each of the spherical IOL (dotted line), the zero aberration IOL (broken line), the aspherical IOL (narrow dotted line), the IOL of the concept of the present invention (I40-O40 is a solid line, I35-O25 is an alternate long and short dash line), in the conditions without the above-described respective changes will be described. This explanation also applies to the subsequent drawings.

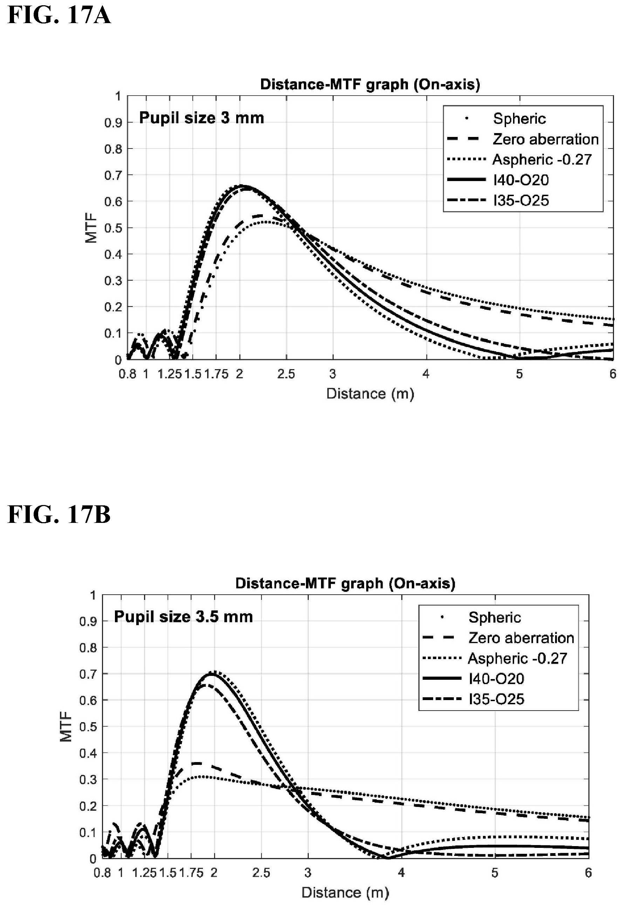

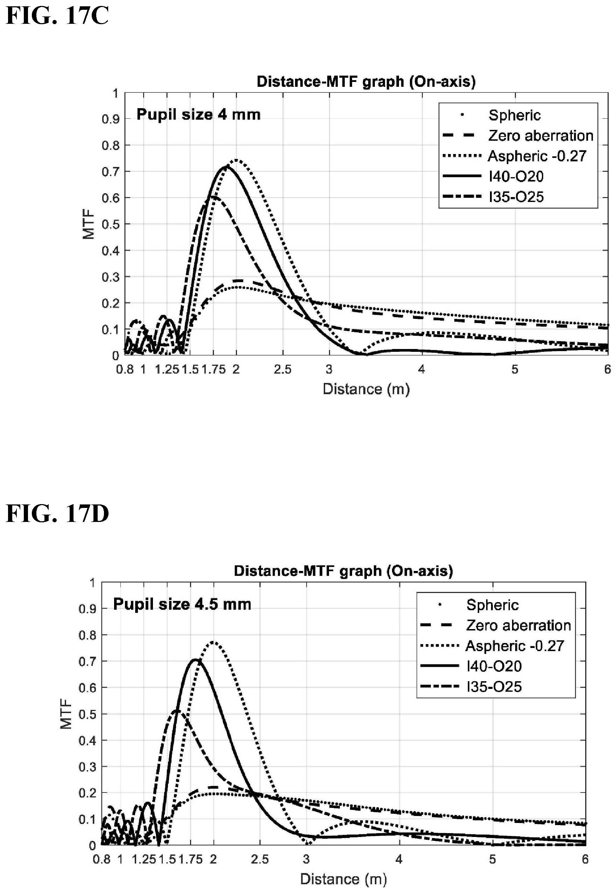

Figure 17 is a plot showing the MTF value (vertical axis) indicating contrast with respect to an object distance (distance) (horizontal axis) from a corneal apex (an aperture diameter (pupil size) is set to 3.0 to 4.5 mm, and no decentration and no tilt).

The above-described plot is obtained by the ZEMAX described above. Note that as for specific contents of the test, WO2008/078804 by the present applicant can be adopted as appropriate.

The MTF (Modulation Transfer Function) value is one of the scales for evaluating lens performance and represents the degree to which the contrast of an object to be viewed can be faithfully reproduced on the image plane, as a spatial frequency characteristic. A higher MTF value means that the contrast perceived by the wearer is higher when looking at an object through the lens.

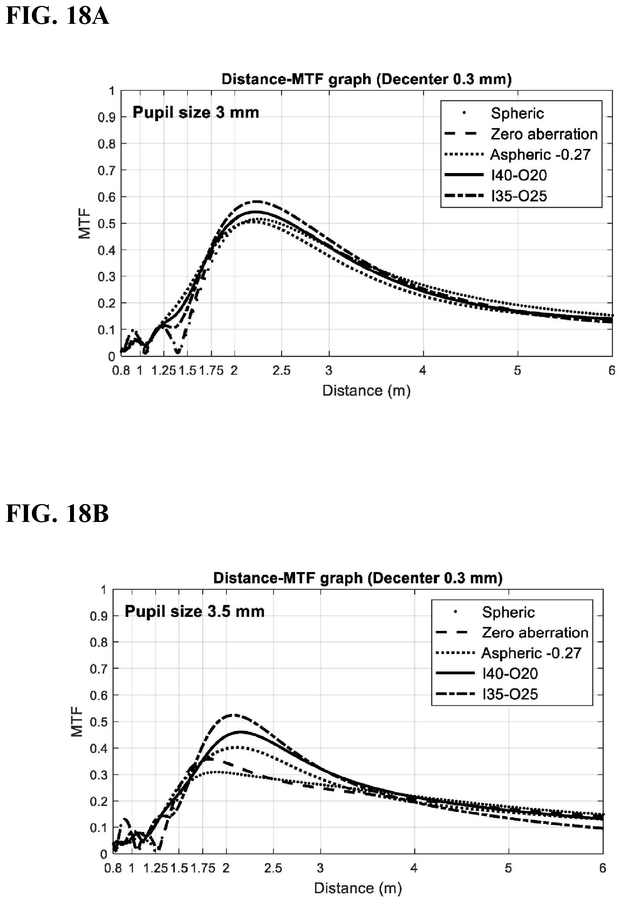

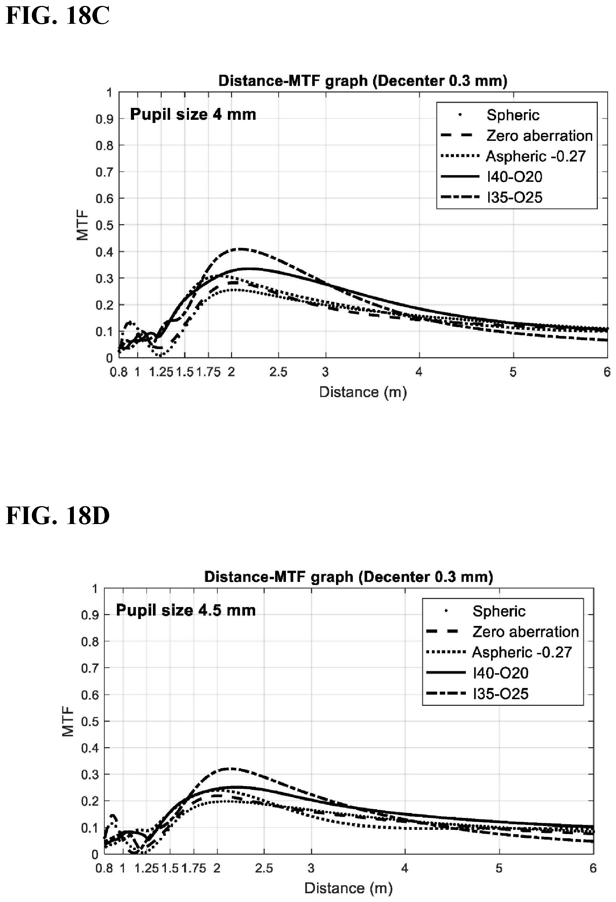

Figure 18 is a plot showing the MTF value (vertical axis) indicating contrast, with respect to the object distance (distance) (horizontal axis) from the corneal apex (aperture diameter (pupil size) is set to 3.0 to 4.5 mm, and decentration is 0.3 mm, no tilt).

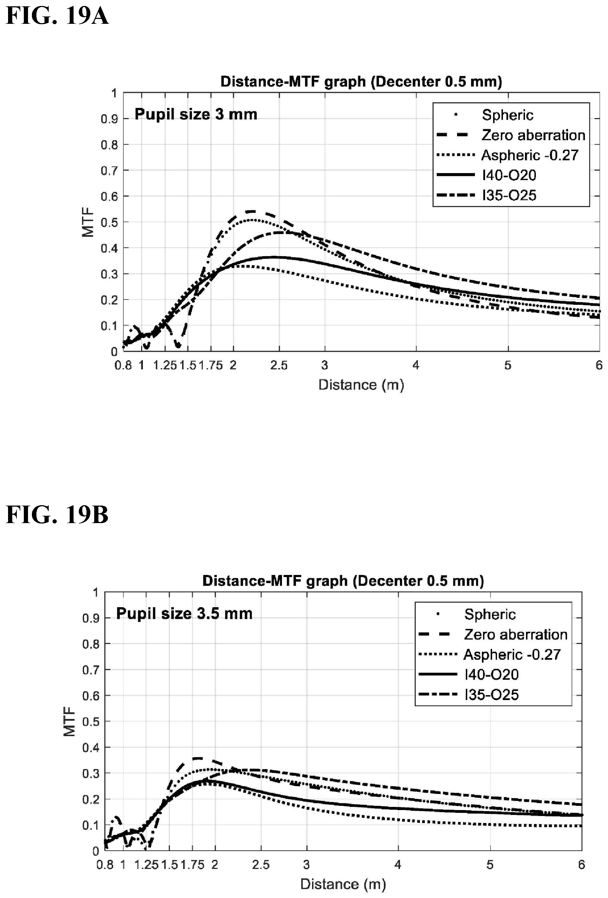

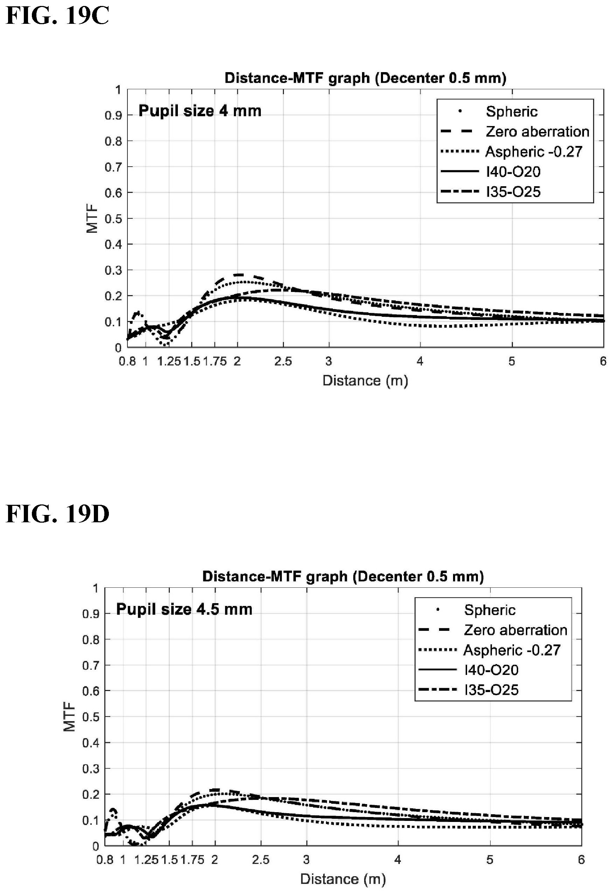

Figure 19 is a plot showing the MTF value (vertical axis) indicating contrast with respect to an object distance (distance) from the corneal apex (horizontal axis) (aperture diameter (pupil size) is set to 3.0 to 4.5 mm, and decentration is 0.5 mm, no tilt).

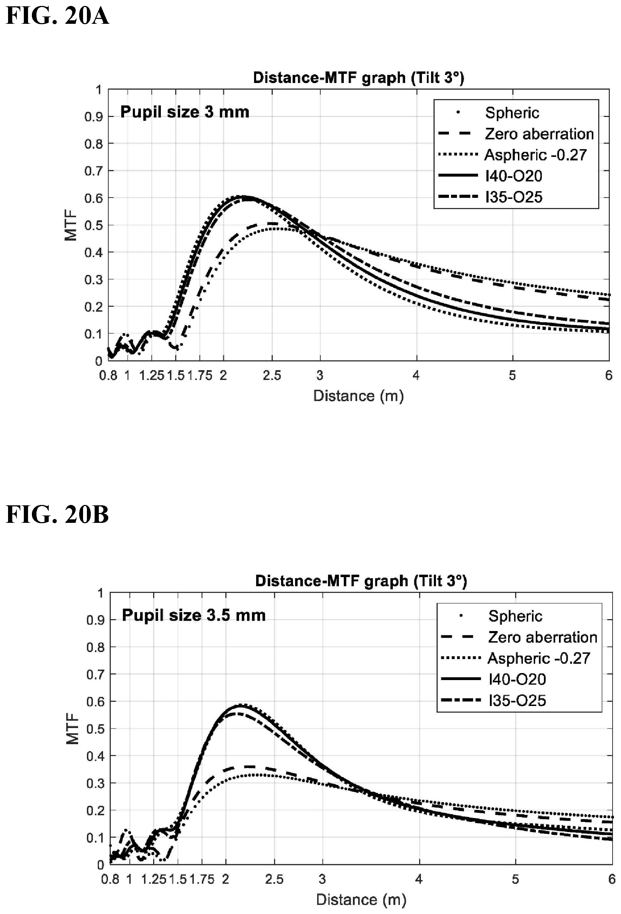

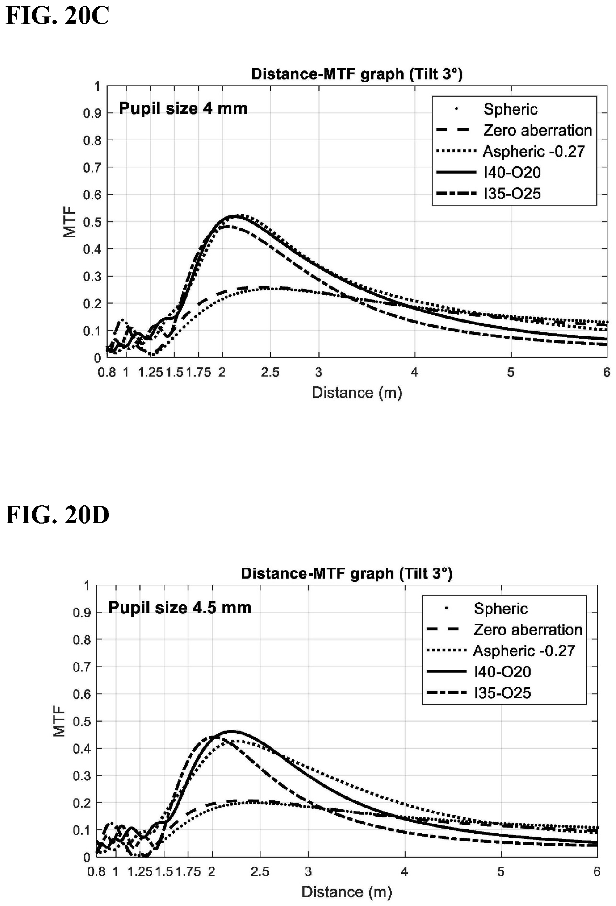

Figure 20 is a plot showing the MTF value (vertical axis) indicating contrast with respect to an object distance (distance) (horizontal axis) from the corneal apex (aperture diameter (pupil size) is set to 3.0 to 4.5 mm, no decentration, and tilt is 3 degrees).

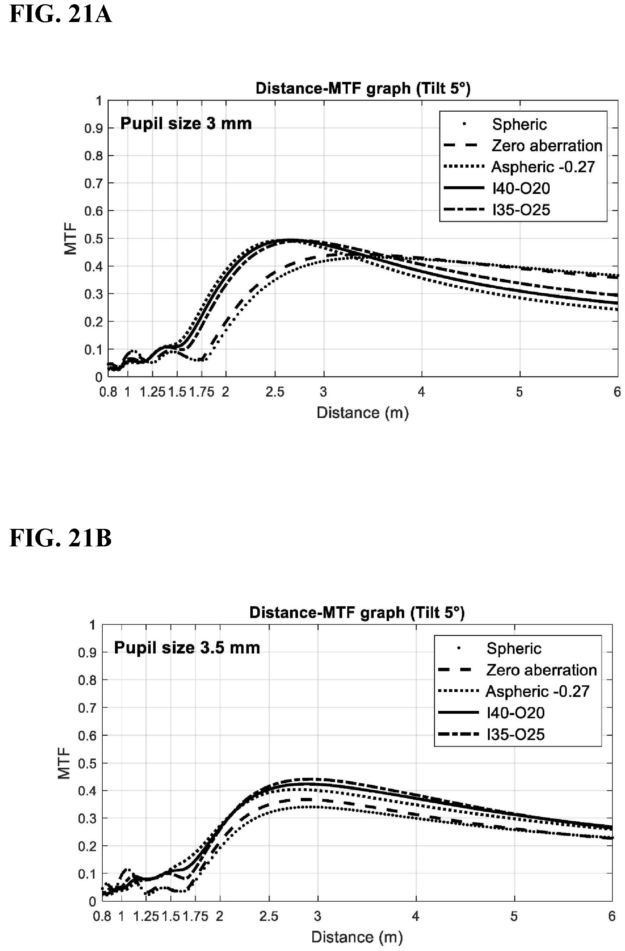

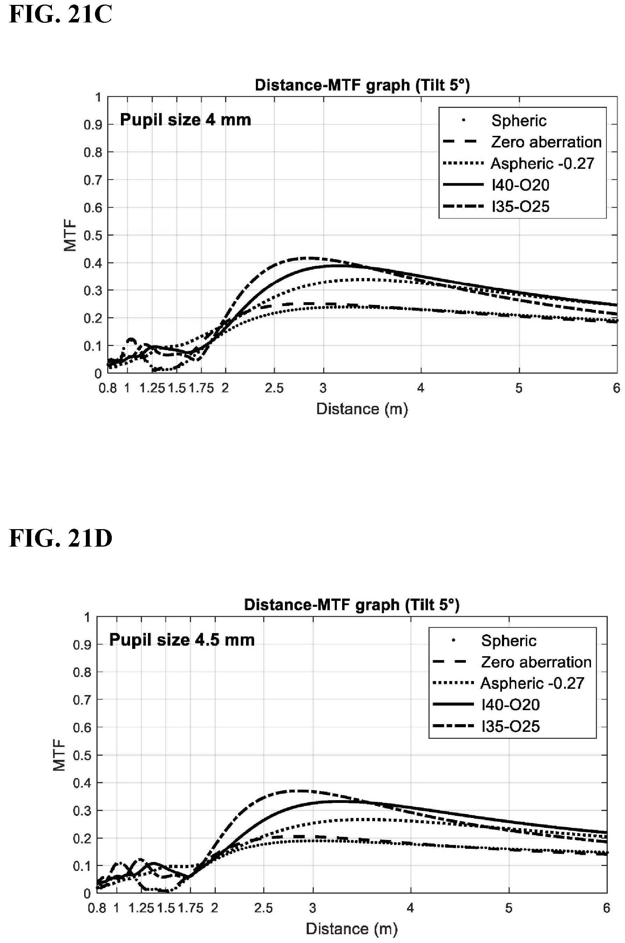

Figure 21 is a plot showing the MTF value (vertical axis) indicating contrast with respect to an object distance (distance) (horizontal axis) from the corneal apex (aperture diameter (pupil size) is set to 3.0 to 4.5 mm, no decentration, and tilt is 5 degrees).

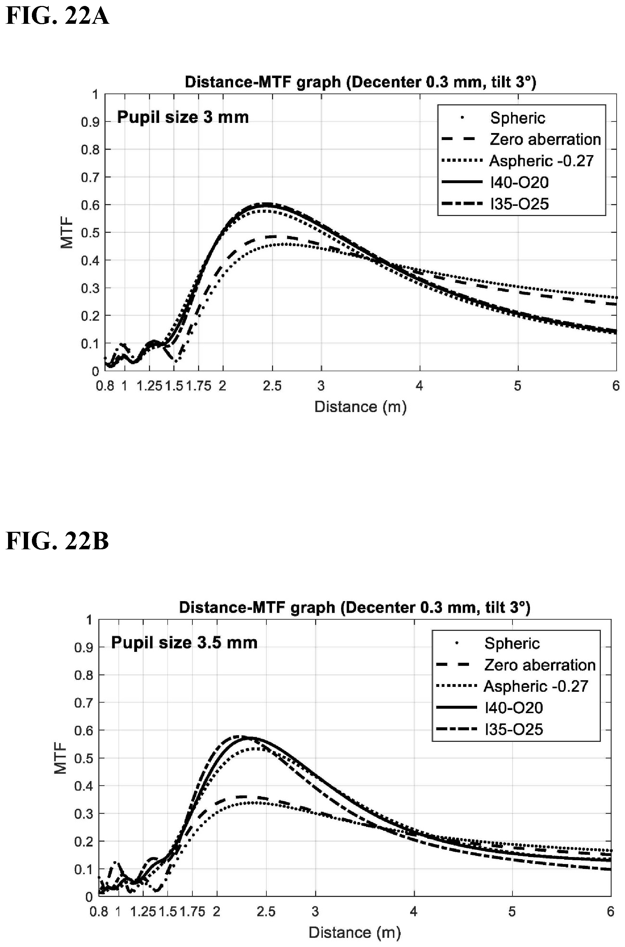

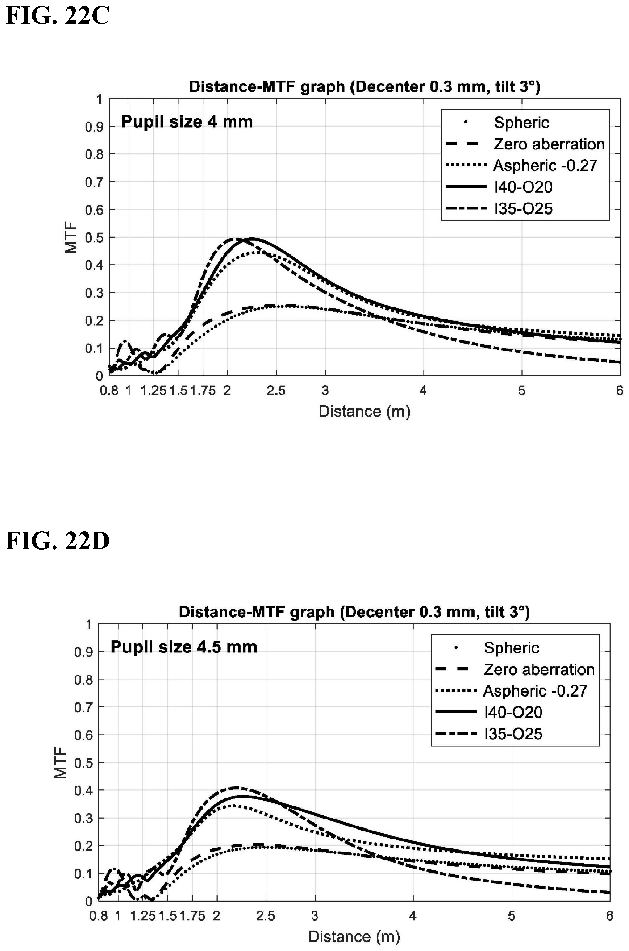

Figure 22 is a plot showing the MTF value (vertical axis) indicating contrast with respect to an object distance (distance) (horizontal axis) from the corneal apex (aperture diameter (pupil size) is set to 3.0 to 4.5 mm, decentration is 0.3 mm, and tilt is 3 degrees).

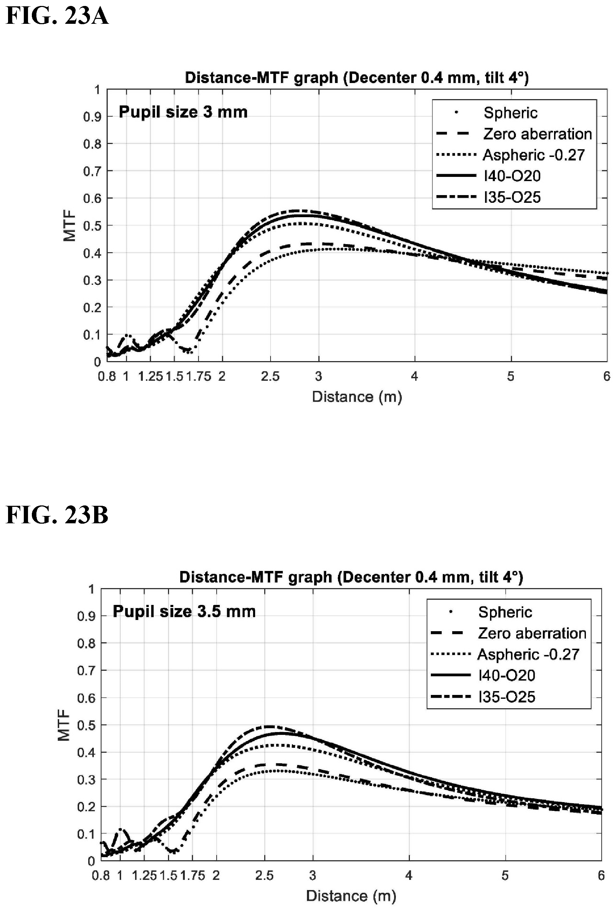

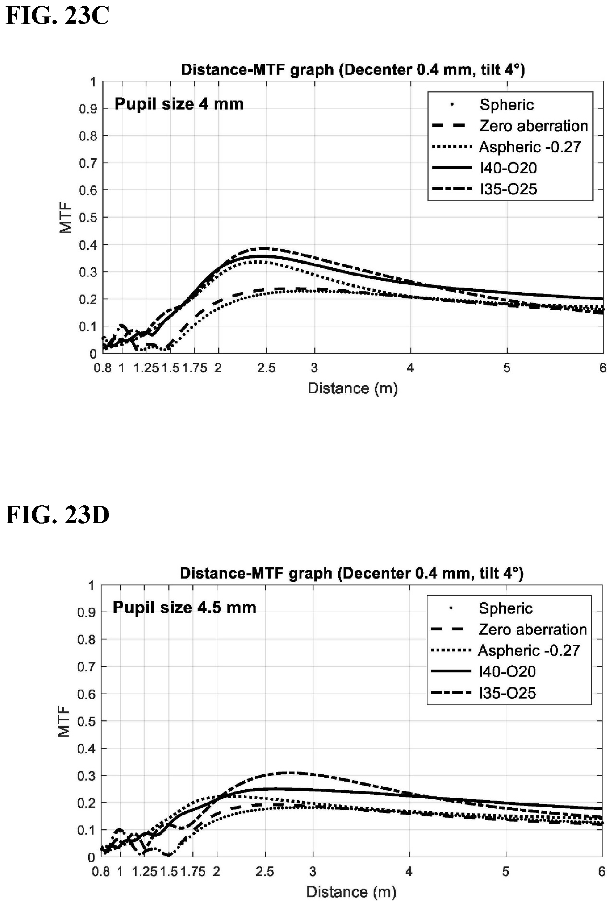

Figure 23 is a plot showing the MTF value (vertical axis) indicating contrast with respect to an object distance (distance) (horizontal axis) from the corneal apex (aperture diameter (pupil size) is set to 3.0 to 4.5 mm, decentration is 0.4 mm, and tilt is 4 degrees).

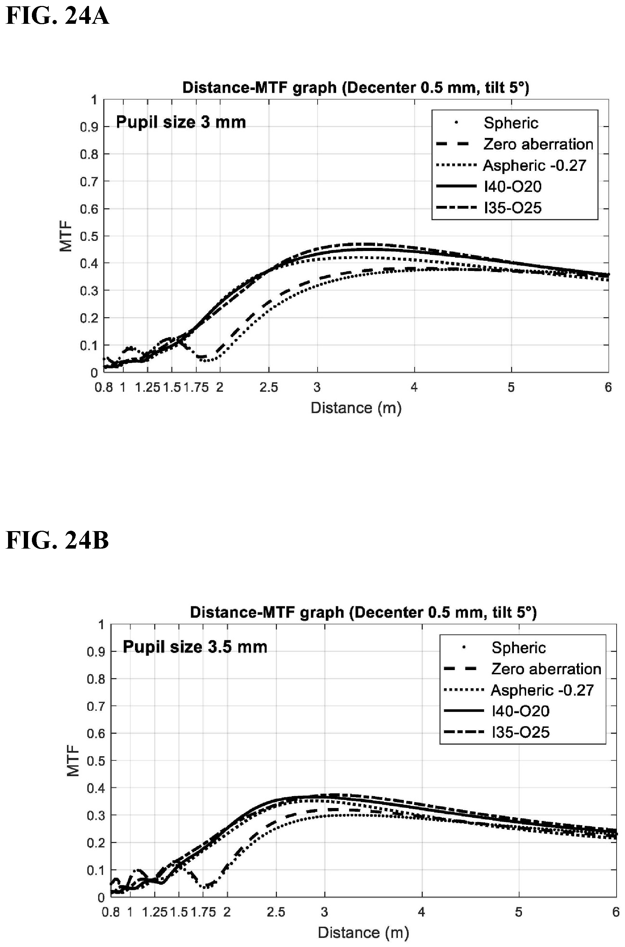

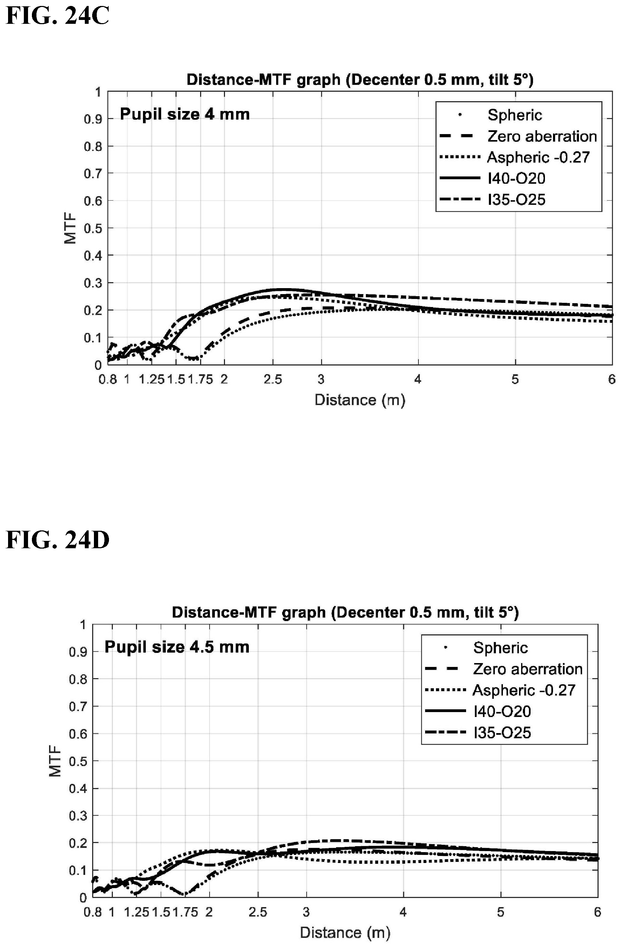

Figure 24 is a plot showing the MTF value (vertical axis) indicating contrast with respect to an object distance (distance) (horizontal axis) from the corneal apex (aperture diameter (pupil size) is set to 3.0 to 4.5 mm, decentration is 0.5 mm, and tilt is 5 degrees).

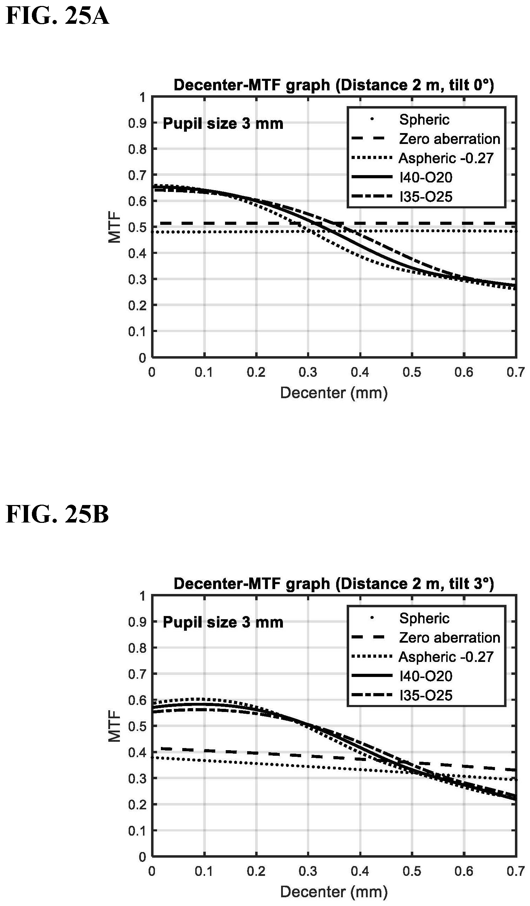

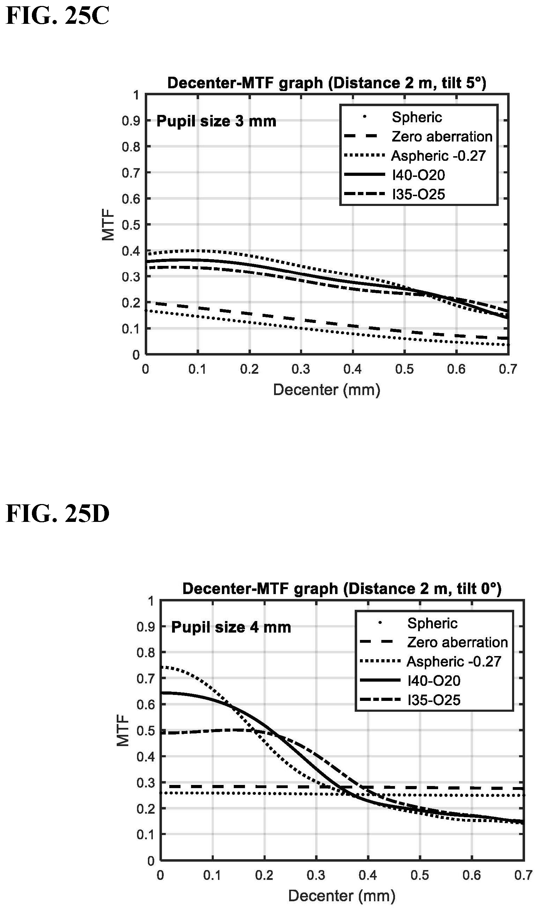

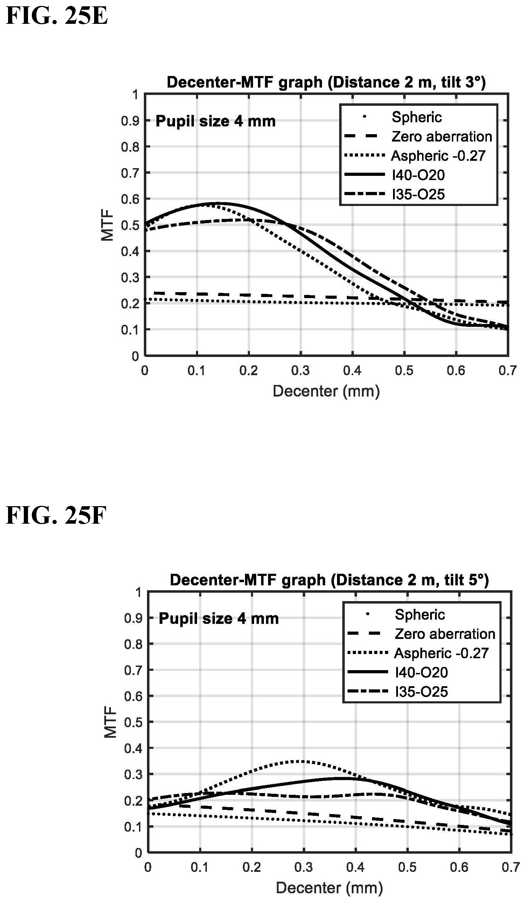

Figure 25 is a plot showing the MTF value (vertical axis) indicating contrast with respect to decentration (horizontal axis) (object distance is set to 2.0 m, aperture diameter (pupil size) is set to 3.0 mm and 4.0 mm, and tilt is 0 to 5 degrees).

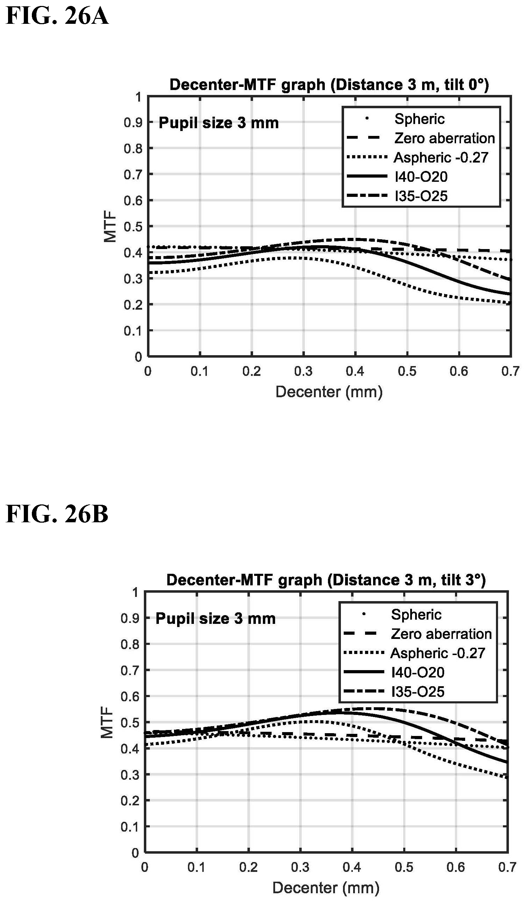

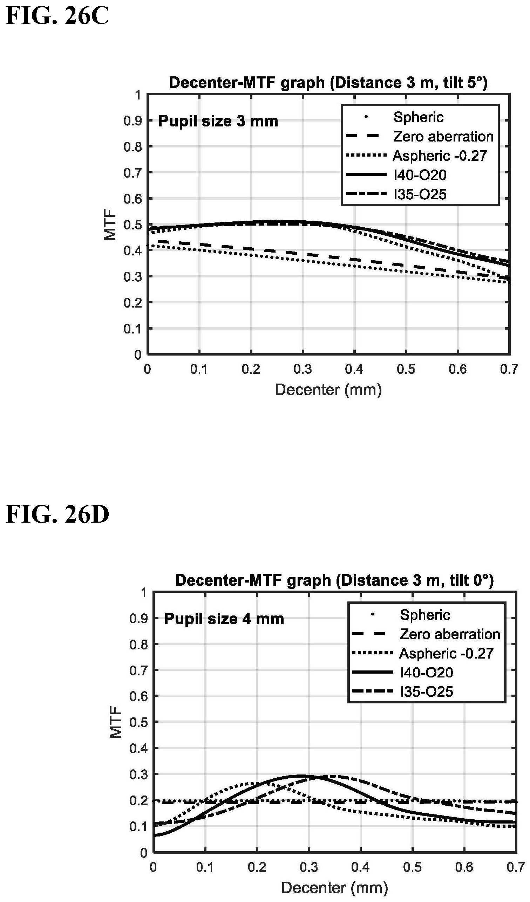

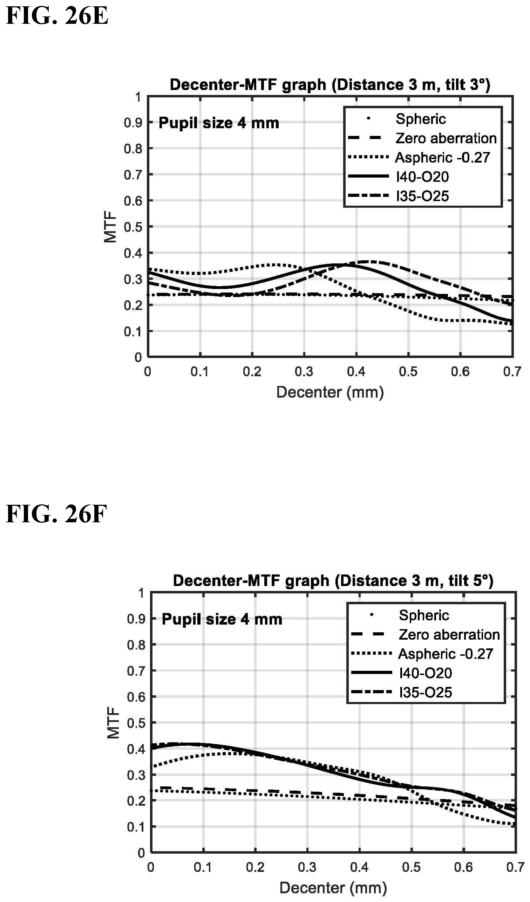

Figure 26 is a plot showing the MTF value (vertical axis) indicating contrast with respect to decentration (horizontal axis) (object distance is set to 3.0 m, aperture diameter (pupil size) is set to 3.0 mm and 4.0 mm, and tilt is 0 to 5 degrees).

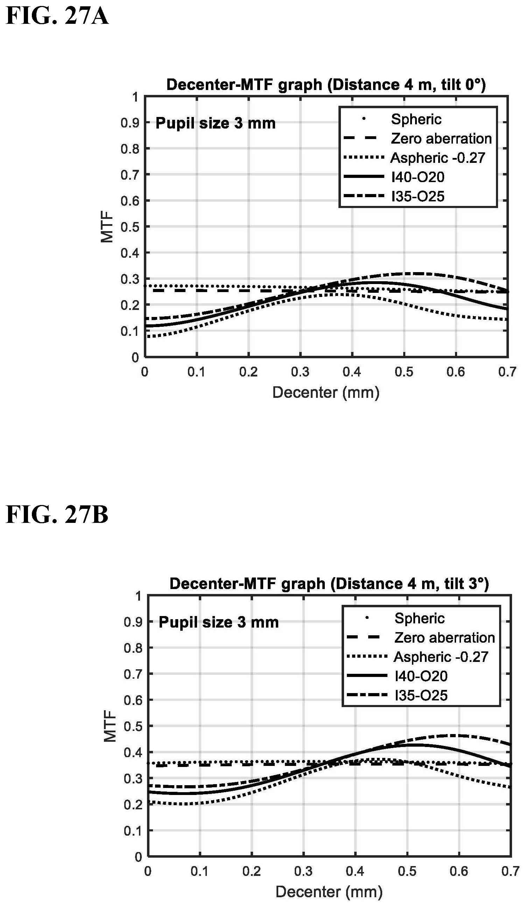

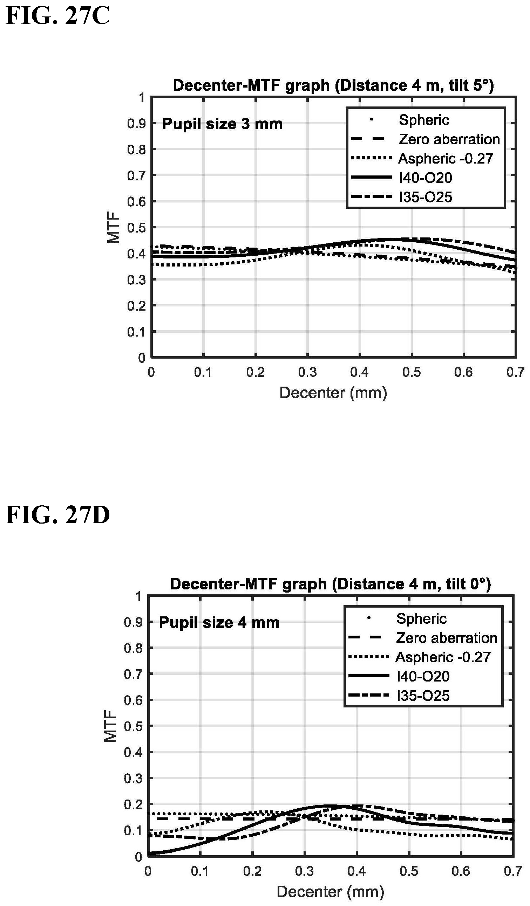

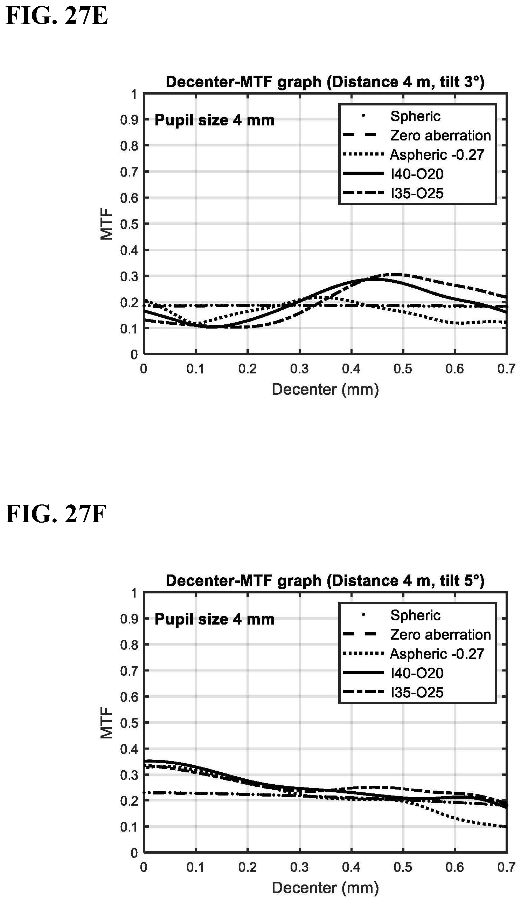

Figure 27 is a plot showing the MTF value (vertical axis) indicating contrast with respect to decentration (horizontal axis) (object distance is set to 4.0 m, aperture diameter (pupil size) is set to 3.0 mm and 4.0 mm, and tilt is 0 to 5 degrees).

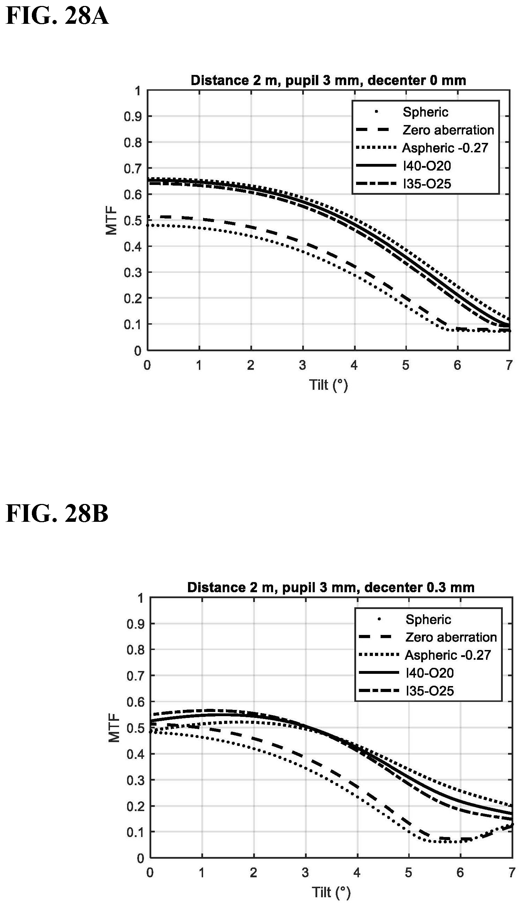

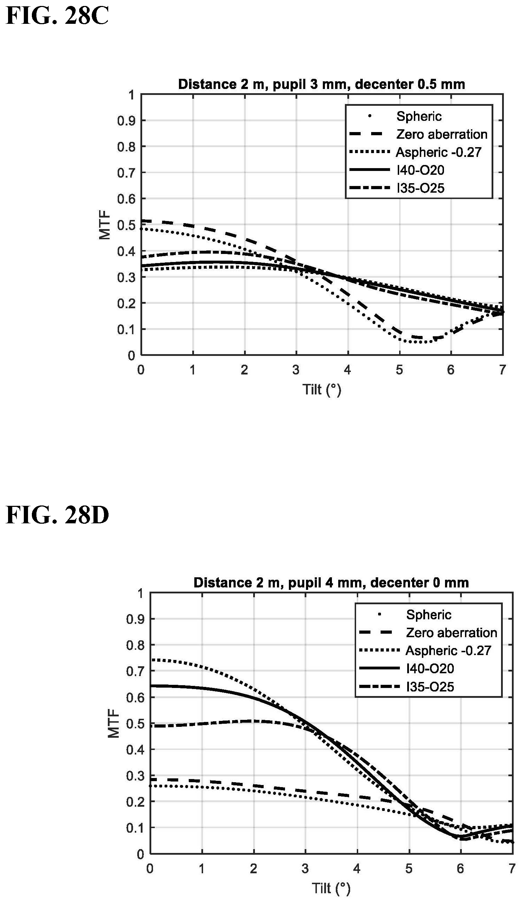

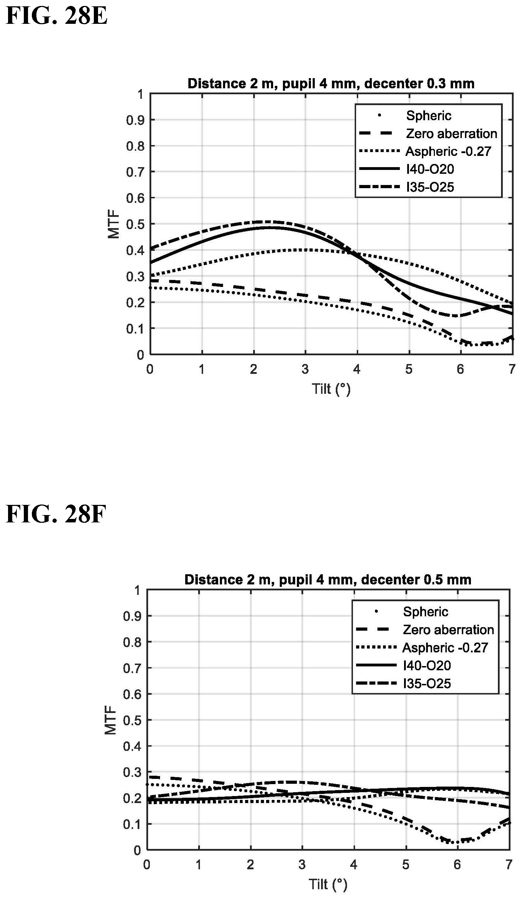

Figure 28 is a plot showing the MTF value (vertical axis) indicating contrast with respect to tilt (horizontal axis) (object distance is set to 2.0 m, aperture diameter (pupil size) is set to 3.0 mm and 4.0 mm, and decentration is 0 to 0.5 mm).

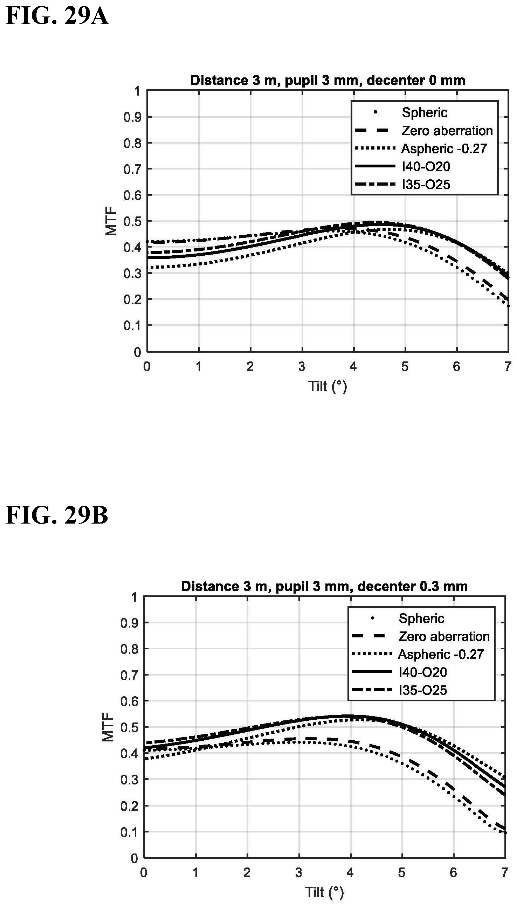

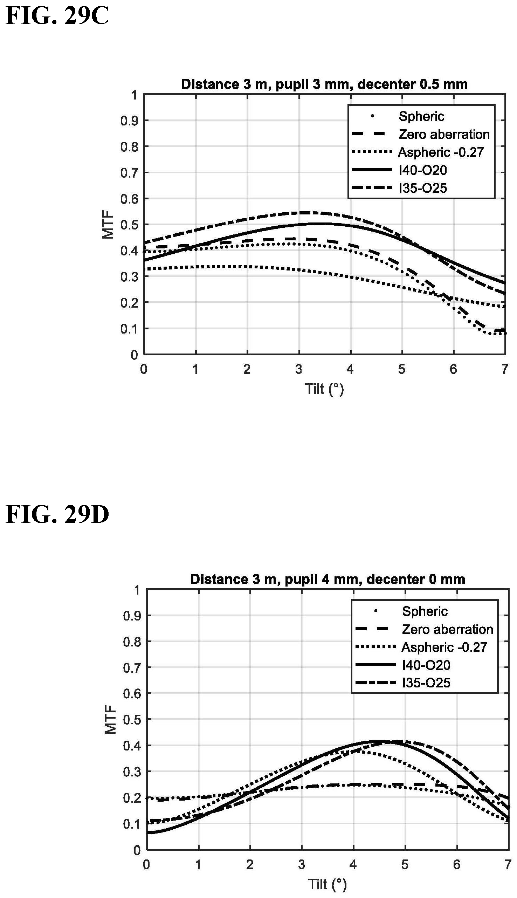

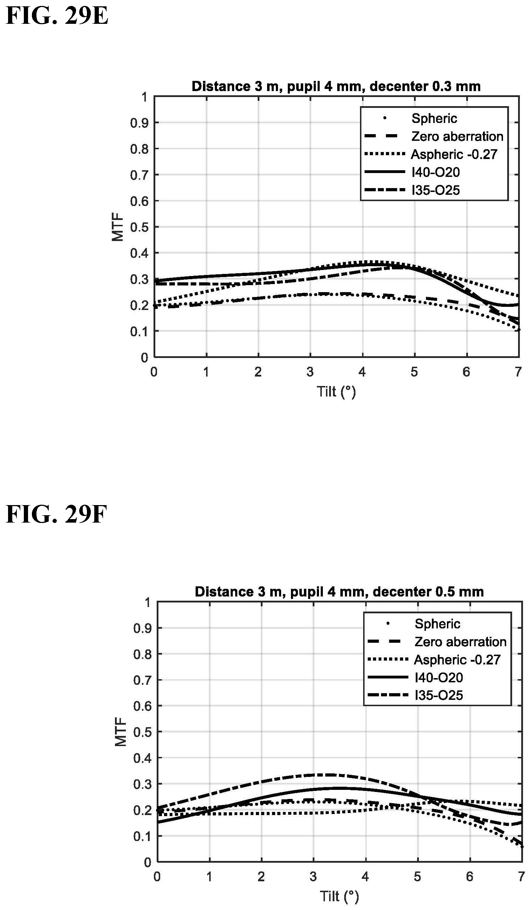

Figure 29 is a plot showing the MTF value (vertical axis) indicating contrast with respect to tilt (horizontal axis) (object distance is set to 3.0 m, aperture diameter (pupil size) is set to 3.0 mm and 4.0 mm, and decentration is 0 to 0.5 mm).

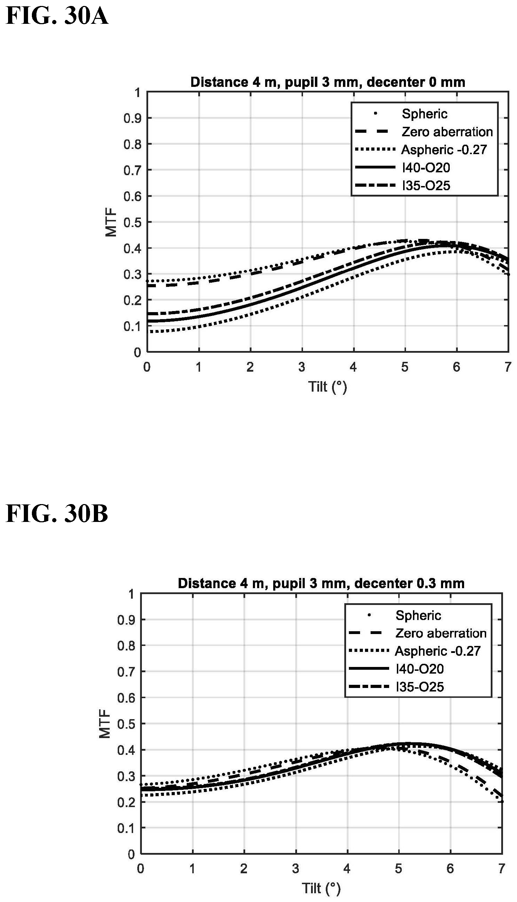

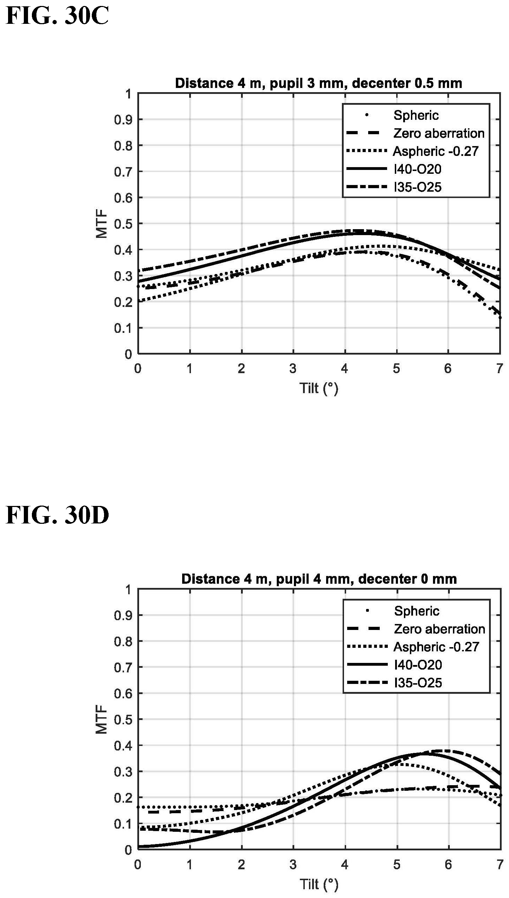

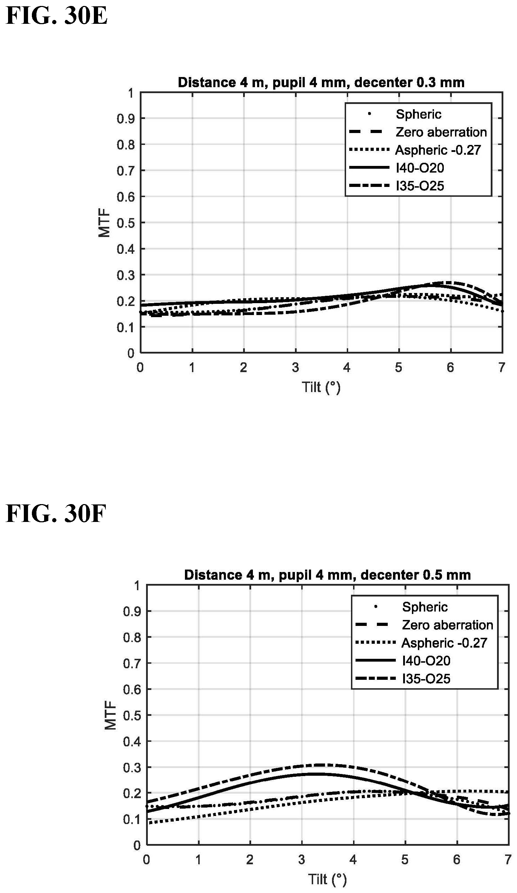

Figure 30 is a plot showing the MTF value (vertical axis) indicating contrast with respect to tilt (horizontal axis) (object distance is set to 4.0 m, aperture diameter (pupil size) is set to 3.0 mm and 4.0 mm, and decentration is 0 to 0.5 mm).

[Embodiment 5]

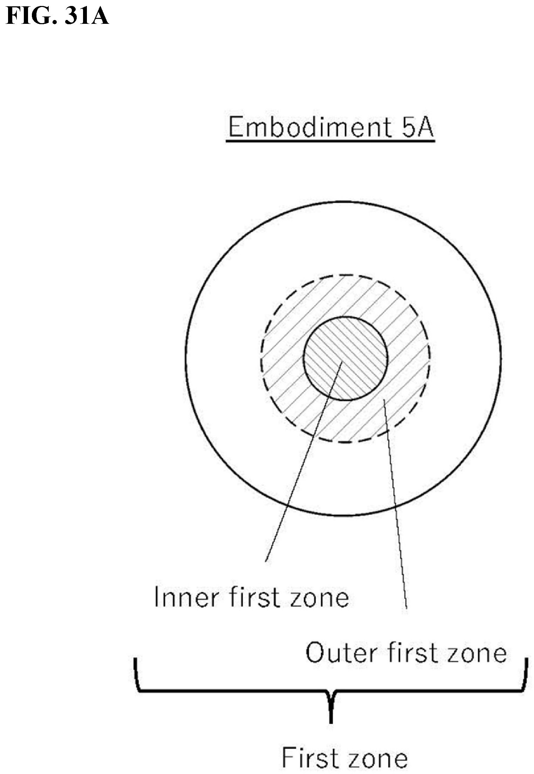

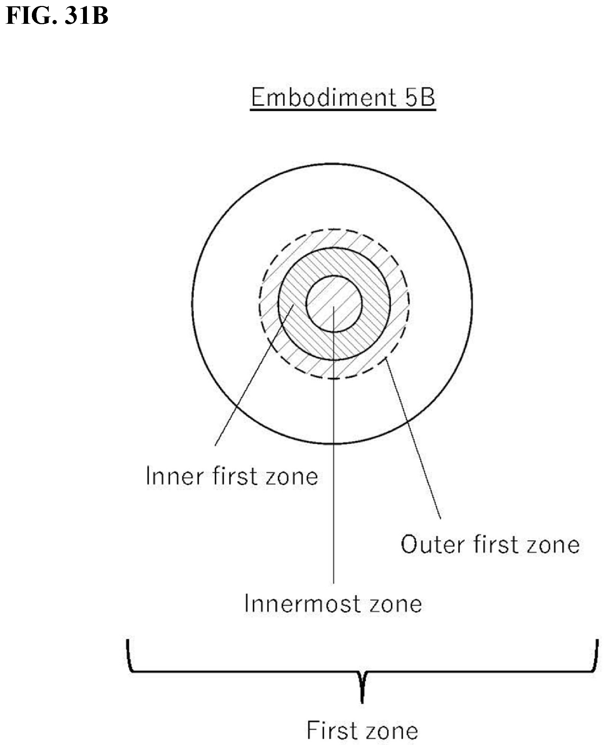

The present invention can also be applied to an intraocular lens in which a positive power is added to at least a part of the power profile in the first zone in the present embodiment to correct near vision and/or intermediate vision in an aphakic patient.
A technical idea (concept) in Embodiment 5 can also be applied to a monofocal lens. For example, it is possible to cite an enhanced monofocal intraocular lens (Enhanced monofocal IOL: commonly known as EM-IOL) having an added positive power (for example, one or more positive constant powers) of 0.75 D or more and 1.75 D or less. Furthermore, the technical idea (concept) in Embodiment 5 can also be applied to a multifocal lens (for example, an added positive power or addition power is 2.5 D or more and 4.0 D or less), and can also be applied to an extended focal depth intraocular lens (extended depth-of-focus IOL: commonly known as EDOF) that is an intermediate lens between a multifocal lens and an EM-IOL (for example, an added positive power is larger than 1.75 D and less than 2.5 D).
A region in which the positive power is added is referred to as an inner first region (Inner First Region). A region that is a region outside thereof (that is present in a direction away from the lens center O), and includes a same power profile Va as the first zone described thus far is referred to as an outer first region (Outer First Region). An example thereof will be described below by using Figure 31 to Figure 34 as Embodiment 5.
The intraocular lens according to Embodiment 5 is as follows.
"An intraocular lens including at least two zones for vision correction that are concentric with a lens center O to which a predetermined base power is set, and adjacent to each other, in which

the zones for vision correction are set as, in order from a first zone including the lens center O outward in a radial direction, the first zone, and a second zone surrounding the first zone, and
in a power profile in which a position seen in the radial direction from the lens center O is set as a horizontal axis (unit: mm), and power is set as a vertical axis (unit: D (diopter)),
when a power profile in a virtual aspherical lens that has a base power in the lens center O and completely cancels positive longitudinal spherical aberration provided by a cornea is an aspherical reference power profile W,
a power profile created by adding values obtained by multiplying values obtained by subtracting vertical axis values in

respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W is a power profile Va for the first zone, and

a power profile created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W is a power profile Vb for the second zone,

the first zone includes an inner first region including a power profile in which a positive power is added to the power profile Va for the first zone,

the second zone includes the power profile Vb, and

an area of the inner first region is less than 50% of a total area of the first zone."

For convenience of explanation, when the lens center O is included in the inner first region, it is referred to as Embodiment 5A, and when it is not (when the lens center O is included in an innermost region shown below that includes a same power profile as the power profile Va) is referred to as Embodiment 5B.

In other words, in Embodiment 5A, as shown in Figure 31A to Figure 33A and Figure 34, the inner first region may include the lens center O.

Further, in Embodiment 5B, as shown in Figure 31B to Figure 33B, the first zone may include an innermost region (Innermost Region) including the same power profile Va as that for the outer first region and including the lens center O, and the inner first region may surround the innermost region.

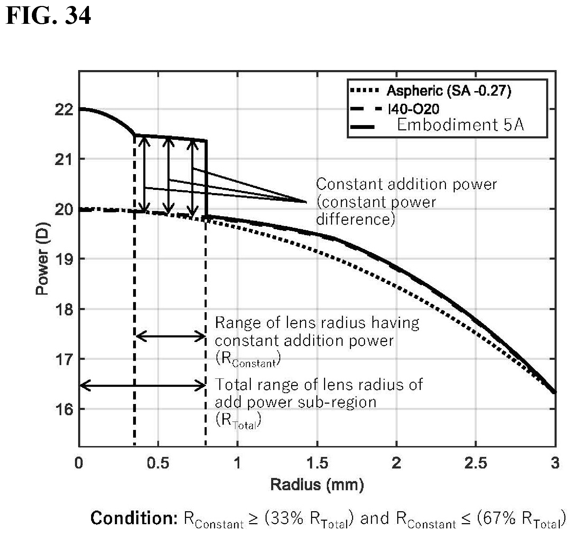

Reference signs A and B in Figure 31 to Figure 34 correspond to Embodiments 5A and 5B. Figure 34 belongs to Embodiment 5A. Note that an embodiment including both the embodiments is referred to as Embodiment 5.

As in each of the above-described embodiments, the first zone is in contact with the second zone in the position ra of the intersection point of the power profiles Va and Vb. As a specific example, the outer first region in the first zone is in contact with the second zone in the position ra of the intersection point.

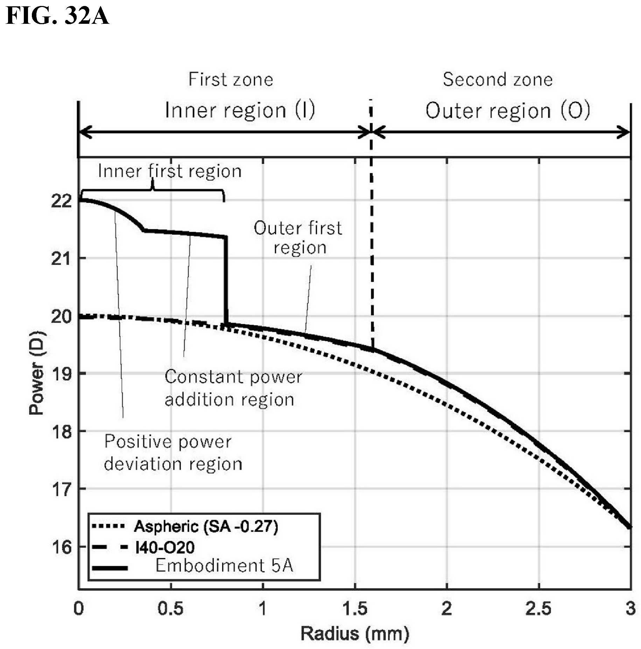

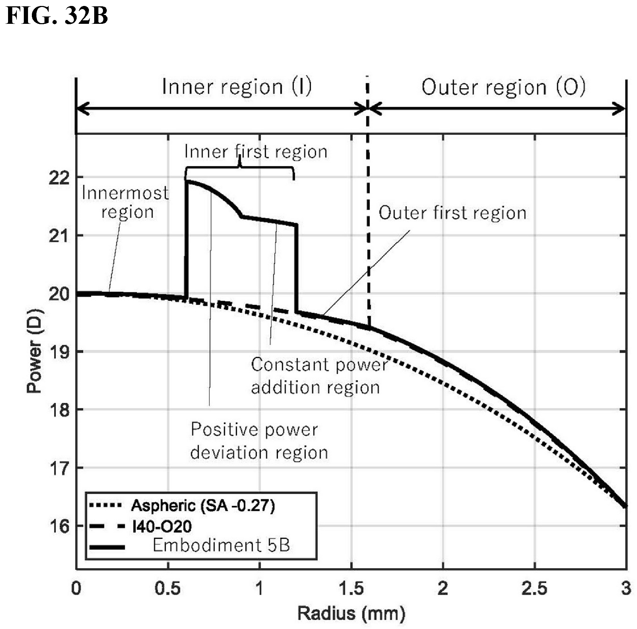

In order to correct near vision and/or intermediate vision of an aphakic patient, an intraocular lens may include an inner first region composed of one or a plurality of regions in the inner region of the optical portion of the IOL of the present invention, as shown in Figure 31A, Figure 31B, Figure 32A, and Figure 32B. An area of the inner first region is less than 50% of the area of the first zone.

The inner first region may include a constant power addition region including a power profile in which a positive constant power is added to the power profile Va for the outer first region.

The "power obtained by adding a positive constant power to the power profile Va" indicates that a deviation from the plot in which the positive constant power is added to the power profile Va is less than $\pm 0.30$ D (preferably, less than $\pm 0.15$ D) in a predetermined distance from the lens center O.

What is added may be other than the positive constant power. A portion to which the positive constant power is added and a portion to which positive non-constant power is added may be provided, and Figure 32A shows an example thereof. Specifically, a positive non-constant power is added to a portion of the power profile from a horizontal axis of 0 mm that is the lens center O to a vicinity of 0.30 mm. In addition, the added power is a power larger than the positive constant power, and in other words, provides a positive power deviation to the positive constant power. This portion is referred to as a positive power deviation region (Positive Power Deviation Region). A positive constant power is added from 0.30 mm to a vicinity of 0.80 mm. This portion is referred to as a constant power addition region (Constant Power Addition Region).

The positive power deviation region may be a power profile that is convex upward as shown in Figure 32A, Figure 33A, and Figure 34, or conversely, may be a power profile that is convex downward. It is preferable that the average value of the absolute values of the differences between the power profile of the positive power deviation region and the power profile in which the positive constant power is added to the power profile Va be larger. In other words, it is preferable that the positive power deviation region be a power profile that is convex upward.

Shapes of the power profiles in the respective regions composing the inner first region may be mutually different shapes. The shapes may be a continuous shape, a discontinuous shape (stepped shape), and a combination of mutually different shapes. Figure 32A and Figure 32B each show a case where different power profiles that are the positive power deviation region and the constant power addition region are adjacently combined.

When seen in the radial direction from the lens center O, a range in the radial direction of the constant power addition region may be 33 to 67% of a range in the radial direction of the inner first region (Figure 34). When the first zone includes a region that is a region other than the inner first region and has an average power equal to the average power of the power profile Va, the average power of the inner first region may be larger than the average power of the region other than the inner first region by 0.75 to 4.0 D, as a specific numeral value of the power. This configuration can provide aphakic patients with clear images of objects at a specific near distance or intermediate distance. As the region in which the average power is equal to the average power of the power profile Va, for example, the above-described outer first region

and/or innermost region can be cited. In the case of this paragraph, even when the outer first region and/or the innermost region are not completely matched with the power profile Va, it is sufficient if the average power includes the above-described relationship. Furthermore, the "region other than the inner first region" may be a region in which a transition region shown below is also excluded in addition to the inner first region. Consistent with this definition, the inner first region may be 0.75 to 4.0 D larger than the average power of the regions other than the inner first region.

The anterior surface, the posterior surface, or both of the surfaces of the lens body of the intraocular lens having two surfaces facing each other may be equipped with the inner first region.

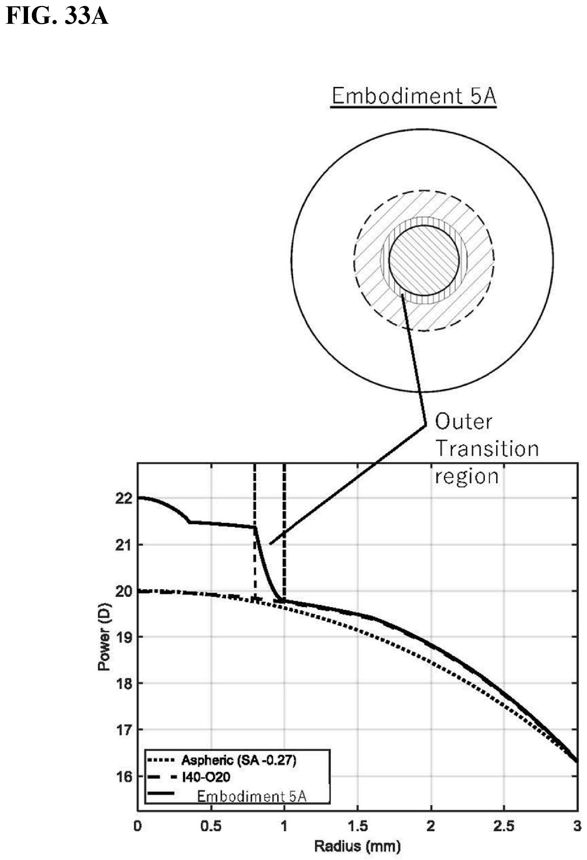

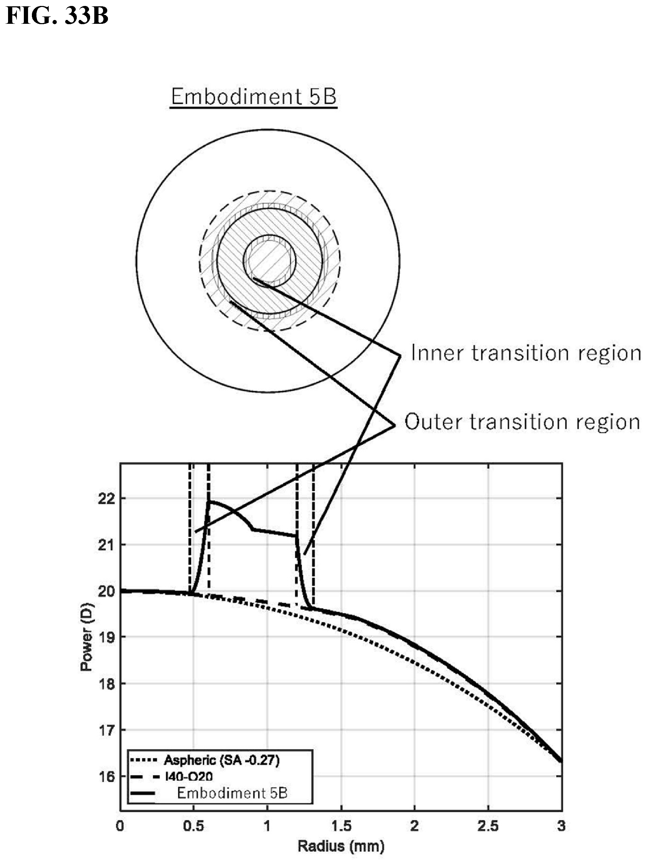

As shown in Figure 33A, in Embodiment 5A, the first zone may include an outer transition region (Outer Transition Region) for smoothly connecting the inner first region to the outer first region. In addition to this, as shown in Figure 33B, in Embodiment 5B, the first zone may include an inner transition region (Inner Transition Region) for smoothly connecting the inner first region to the innermost region. In both the transition regions, the power decreases toward the power profile Va, in other words, a negative power deviation is brought about to the positive constant power described above.

Both the transition regions may have power profiles that are convex downward as shown in Figure 33A and Figure 33B, or conversely may have power profiles that are convex upward. It is preferable that the average value of the absolute values of the differences between the power profile of the positive power deviation region and the power profile in which the positive constant power is added to the power profile Va be small. In other words, it is preferable that the positive power deviation region have a power profile that is convex downward.

In the present invention, the case where the outer first region is not provided is also allowed. In other words, the inner first region (example: constant power addition region) or the outer transition region may be provided to contact the position ra of the intersection point of the power profiles Va and Vb. In this case, the average power in the first zone becomes high, and therefore near vision and intermediate vision are enhanced.

The anterior surface, the posterior surface, or both of them of the lens body of the intraocular lens having two surfaces facing each other may be aspherical.

The anterior surface, the posterior surface, or both of them of the lens body of the intraocular lens having two surfaces facing each other may include a toric surface including a cylinder power for astigmatism correction for an aphakic patient.

## Claims

1. An intraocular lens comprising at least two zones for vision correction that are concentric with a lens center O to which a predetermined base power is set and adjacent to each other,

    wherein when the zones for vision correction are set as, in order from a first zone including the lens center O outward in a radial direction, the first zone, and a second zone surrounding the first zone, and
    a position of a first boundary between the first zone and the second zone when seen in the radial direction from the lens center O is r1, in a power profile in which a position when seen in the radial direction from the lens center O is set as a horizontal axis (unit: mm), and power is set as a vertical axis (unit: D (diopter)), and
    when a power profile in a virtual aspherical lens that has a base power in the lens center O and completely cancels positive longitudinal spherical aberration provided by a cornea is an aspherical reference power profile W,
    the intraocular lens comprises:

        a power profile V that is included within a region of a set of power profiles obtained by respective combinations of power profiles Va and Vb when an intersection point of
        the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W, and
        the power profile Vb for the second zone created by adding values obtained by multiplying values obtained by subtracting the horizontal axis values in the respective vertical axis values of the aspherical reference power profile W from a maximum value rmax of the horizontal axis of the aspherical reference power profile W by a predetermined ratio $\beta$ ($\beta$ is 10% or more and 50% or less) to the horizontal axis values in the respective vertical axis values of the aspherical reference power profile W

    is a position ra in a horizontal axis.

2. The intraocular lens according to claim 1, wherein r1 is a value within a range of 1.5 mm or more and 2.3 mm or less.

**3.** The intraocular lens according to claim 1, wherein the position r1 has a positive maximum value among values obtained by subtracting the vertical axis values of the aspherical reference power profile W from vertical axis values of the power profile V, in respective horizontal axis values of the power profile V.

**4.** The intraocular lens according to claim 1, wherein a maximum value rmax of a horizontal axis of the power profile V is a value within a range of 2.5 mm or more and 3.5 mm or less.

**5.** The intraocular lens according to claim 1, comprising

one or more additional regions from the second zone outward in the radial direction,
wherein the additional regions surround the second zone, and
in respective horizontal axis values of a power profile Vadd in at least one region of the additional regions, values obtained by subtracting the vertical axis values of the aspherical reference power profile W from vertical axis values of the power profile Vadd are less than $\pm 0.30$ D.

**6.** The intraocular lens according to claim 1, wherein the power profile V is expressed by a polynomial expression.

**7.** The intraocular lens according to claim 1, comprising

one or more additional regions from the second zone outward in the radial direction,
wherein the additional regions surround the second zone, and
the additional regions include a function of refracting incident light beams onto a retina.

**8.** An intraocular lens comprising at least two zones for vision correction that are concentric with a lens center O to which a predetermined base power is set and adjacent to each other,

wherein when the zones for vision correction are set as, in order from a first zone including the lens center O outward in a radial direction, the first zone, and a second zone surrounding the first zone, and
a position of a first boundary between the first zone and the second zone when seen in the radial direction from the lens center O is r1, in a power profile in which a position seen in the radial direction from the lens center O is set as a horizontal axis (unit: mm), and a power is set as a vertical axis (unit: D (diopter)), and
when a power profile in a virtual aspherical lens that has a base power in the lens center O and completely cancels positive longitudinal spherical aberration provided by a cornea is an aspherical reference power profile W,
in the first zone, an average value of vertical axis values of a power profile V1 for the first zone is larger than an average value of vertical axis values of the aspherical reference power profile W,
in the second zone, an average value of vertical axis values of a power profile V2 for the second zone is larger than the average value of the vertical axis values of the aspherical reference power profile W,
in the power profile V1 for the first zone, with increase in a horizontal axis value, a value obtained by subtracting a vertical axis value of the aspherical reference power profile W from a vertical axis value of the power profile V1 continuously increases in each of horizontal axis values,
in the power profile V2 for the second zone, with increase in a horizontal axis value, a value obtained by subtracting the vertical axis value of the aspherical reference power profile W from a vertical axis value of the power profile V2 continuously decreases in each of horizontal axis values,
in a power profile V, the position r1 has a positive maximum value among values obtained by subtracting the vertical axis values of the aspherical reference power profile W from the vertical axis values of the power profile V2, and
a value obtained by subtracting a vertical axis value of the aspherical reference power profile W from a vertical axis value in a maximum value rmax of a horizontal axis of the power profile V is less than 0.25 D.

**9.** The intraocular lens according to claim 8, wherein an absolute value of an average value of a slope of a tangent line of the power profile V2 in the second zone is equal to or more than three times an absolute value of an average value of a slope of a tangent line of the power profile V1 in the first zone.

**10.** The intraocular lens according to claim 8, wherein rl is a value within a range of 1.5 mm or more and 2.3 mm or less.

**11.** The intraocular lens according to claim 8, wherein the maximum value rmax of the horizontal axis of the power profile V is a value within a range of 2.5 mm or more and 3.5 mm or less.

**12.** The intraocular lens according to claim 8, comprising the power profile V that is included within a region of a set of power profiles obtained by respective combinations of power profiles Va and Vb, when an intersection point of

the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W, and
the power profile Vb for the second zone created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W

is a position ra in a horizontal axis.

**13.** The intraocular lens according to claim 8, comprising:

one or more additional regions from the second zone outward in the radial direction,
wherein the additional regions surround the second zone,
in respective horizontal axis values of a power profile Vadd in at least one region of the additional regions, values obtained by subtracting the vertical axis values of the aspherical reference power profile W from vertical axis values of the power profile Vadd are less than $\pm$0.30 D.

**14.** The intraocular lens according to claim 8, wherein the power profile V is expressed by a polynomial expression.

**15.** The intraocular lens according to claim 8, comprising one or more additional regions from the second zone outward in the radial direction,

wherein the additional regions surround the second zone, and
the additional regions include a function of refracting incident light beams onto a retina.

**16.** An intraocular lens comprising at least two zones for vision correction that are concentric with a lens center O to which a predetermined base power is set and adjacent to each other,

wherein when the zones for vision correction are set as, in order from a first zone including the lens center O outward in a radial direction, the first zone, a middle zone surrounding the first zone, and a second zone surrounding the middle zone, and
in a power profile in which a position when seen in the radial direction from the lens center O is set as a horizontal axis (unit: mm), and a power is set as a vertical axis (unit: D (diopter)),
a power profile in a virtual aspherical lens that has a base power in the lens center O and completely cancels positive longitudinal spherical aberration provided by a cornea is an aspherical reference power profile W,
the intraocular lens comprises a power profile V that is included within a region of a set of power profiles obtained by respective combinations of power profiles Va, Vmid and Vb, configured by
the power profile Va for the first zone created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal axis values of the aspherical reference power profile W,
the power profile Vb for the second zone created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W, and
the power profile Vmid for the middle zone in which an average power is smaller than an average power in the first zone, and larger than an average power in the second zone.

**17.** The intraocular lens according to claim 16, wherein an absolute value of an average value of a slope of a tangent line of the power profile Vmid in the middle zone is larger than an absolute value of an average value of a slope of a tangent line of a power profile V1 in the first zone, and is smaller than an absolute value of an average value of a slope of a

tangent line of a power profile V2 in the second zone.

**18.** The intraocular lens according to claim 16,

wherein in respective horizontal axis values of the power profile Vmid in the middle zone, an average value of values obtained by subtracting the vertical axis values of the aspherical reference power profile W from vertical axis values of the power profile Vmid is
larger than an average value of values obtained by subtracting the vertical axis values of the aspherical reference power profile W from vertical axis values of a power profile V1 in respective horizontal axis values of the power profile V1 in the first zone, and
larger than an average value of values obtained by subtracting the vertical axis values of the aspherical reference power profile W from vertical axis values of a power profile V2 in respective horizontal axis values of the power profile V2 in the second zone.

**19.** The intraocular lens according to claim 16, wherein in the middle zone, horizontal axis values are included within a range of 1.3 mm or more and 2.5 mm or less.

**20.** The intraocular lens according to claim 16, wherein a maximum value rmax of a horizontal axis of the power profile V is a value within a range of 2.5 mm or more and 3.5 mm or less.

**21.** The intraocular lens according to claim 16, wherein an absolute value of an average value of a slope of a tangent line of a power profile V2 in the second zone is equal to or larger than three times an absolute value of an average value of a slope of a tangent line of a power profile V1 in the first zone.

**22.** The intraocular lens according to claim 16, comprising one or more additional regions outward in the radial direction from the second zone,

wherein the additional regions surround the second zone, and
in respective horizontal axis values of a power profile Vadd in at least one region of the additional regions, values obtained by subtracting the vertical axis values of the aspherical reference power profile W from vertical axis values of the power profile Vadd are less than $\pm$0.30 D.

**23.** The intraocular lens according to claim 16, wherein the power profile V is expressed by a polynomial expression.

**24.** The intraocular lens according to claim 16, comprising one or more additional regions outward in the radial direction from the second zone,

wherein the additional regions surround the second zone, and
the additional regions include a function of refracting incident light beams onto a retina.

**25.** An intraocular lens comprising at least two zones for vision correction that are concentrical with a lens center O to which a predetermined base power is set and adjacent to each other,

wherein when the zones for vision correction are set as, in order from a first zone including the lens center O outward in a radial direction, the first zone, and a second zone surrounding the first zone, and
a power profile in a virtual aspherical lens that has a base power in the lens center O and completely cancels positive longitudinal spherical aberration provided by a cornea is an aspherical reference power profile W, and when a position of a first boundary between the first zone and the second zone seen in the radial direction from the lens center O is r1, in a total power profile in which a position when seen in the radial direction from the lens center O is set as a horizontal axis (unit: mm), a total power T when refractive power of the cornea and power of the intraocular lens are added up is set as a vertical axis (unit: D (diopter)),
the intraocular lens comprises a total power profile TV that is included within a region of a set of total power profiles obtained by respective combinations of total power profiles TVa and TVb, when an intersection point of
the total power profile TVa for the first zone created by adding values obtained by multiplying values obtained by subtracting vertical axis values in respective horizontal axis values of an aspherical reference total power profile TW in which the refractive power of the cornea is added from the base power by a predetermined ratio $\alpha$ ($\alpha$ is 10% or more and 50% or less) to the vertical axis values in the respective horizontal axis values of the aspherical reference total power profile TW, and

the total power profile TVb for the second zone created by adding values obtained by multiplying values obtained by subtracting the horizontal axis values in the respective vertical axis values of the aspherical reference total power profile TW in which the refractive power of the cornea is added from a maximum value rmax in a horizontal axis of the aspherical reference total power profile TW by a predetermined ratio $\beta$ ($\beta$ is 10% or more and 50% or less) to the horizontal axis values in the respective vertical axis values of the aspherical reference total power profile TW

is a position ra in a horizontal axis.

**26.** The intraocular lens according to claim 25, wherein

in a total power profile TV1 for the first zone, with increase in a horizontal axis value, a vertical axis value continuously increases, and
in a total power profile TV2 for the second zone, with increase in the horizontal axis value, the vertical axis value continuously decreases.

**27.** The intraocular lens according to claim 25,

wherein an absolute value of an average value of a slope of a tangent line of a total power profile TV1 in a vicinity of an origin in the first zone is smaller than an absolute value of an average value of a slope of a tangent line of the total power profile TV1 in a vicinity of a middle position in the first zone,
the absolute value of the average value of the slope of the tangent line of the total power profile TV1 in the vicinity of the middle position in the first zone is larger than an absolute value of an average value of a slope of a tangent line of the total power profile TV1 in a vicinity of the position r1 in the first zone, and
the absolute value of the average value of the slope of the tangent line of the total power profile TV1 in the vicinity of the position r1 in the first zone is smaller than an absolute value of an average value of a slope of a tangent line of a total power profile TV2 in a vicinity of a middle position in the second zone.

**28.** The intraocular lens according to claim 25,

wherein in the total power profile TV, a value obtained by subtracting a vertical axis value in r=0 from a vertical axis value in the position rl is a positive maximum value, and
r1 is a value within a range of 1.5 mm or more and 2.3 mm or less.

**29.** The intraocular lens according to claim 25, wherein a value obtained by subtracting a vertical axis value in r=0 from a vertical axis value in a maximum value rmax of a horizontal axis of the total power profile TV is less than $\pm$0.30 D.

**30.** The intraocular lens according to claim 25, wherein a maximum value rmax of a horizontal axis of the total power profile TV is a value within a range of 2.5 mm or more and 3.5 mm or less.

**31.** The intraocular lens according to claim 25, comprising one or more additional regions from the second zone outward in the radial direction,

wherein the additional regions surround the second zone, and
the additional regions include a function of refracting incident light beams onto a retina.

**32.** An intraocular lens comprising at least two zones for vision correction that are concentric with a lens center O to which a predetermined base power is set and adjacent to each other,

wherein when the zones for vision correction are set as, in order from a first zone including the lens center O outward in a radial direction, the first zone, and a second zone surrounding the first zone,
in a power profile in which a position when seen in the radial direction from the lens center O is set as a horizontal axis (unit: mm), and a power is set as a vertical axis (unit: D (diopter)),
a power profile in a virtual aspherical lens that has a base power in the lens center O and completely cancels positive longitudinal spherical aberration provided by a cornea is an aspherical reference power profile W,
a power profile created by adding values obtained by multiplying values obtained by subtracting vertical axis values in respective horizontal axis values of the aspherical reference power profile W from the base power by a predetermined ratio $\alpha$ ($\alpha$ is 10% or more and 50% or less) to the vertical axis values in the respective horizontal

axis values of the aspherical reference power profile W is a power profile Va for the first zone, and
a power profile created by adding values obtained by multiplying values obtained by subtracting the horizontal axis values in the respective vertical axis values of the aspherical reference power profile W from a maximum value rmax of a horizontal axis of the aspherical reference power profile W by a predetermined ratio $\beta$ ($\beta$ is 10% or more and 50% or less) to the horizontal axis values in the respective vertical axis values of the aspherical reference power profile W is a power profile Vb for the second zone,
the first zone includes an inner first region including a power profile in which a positive power is added to the power profile Va for the first zone,
the second zone includes the power profile Vb, and
an area of the inner first region is less than 50% of a total area of the first zone.

**33.** The intraocular lens according to claim 32,

wherein the first zone incudes the inner first region, and an outer first region surrounding the inner first region, when seen in the radial direction from the lens center O, and
the outer first region includes the power profile Va, and is in contact with the second zone in a position of an intersection point of the power profiles Va and Vb.

**34.** The intraocular lens according to claim 32, wherein an anterior surface, a posterior surface, or both of them of a lens body of the intraocular lens having two surfaces facing each other is or are equipped with the inner first region.

**35.** The intraocular lens according to claim 32, wherein the first zone includes a region that is other than the inner first region, and has an average power equal to an average power of the power profile Va, and an average power of the inner first region is larger than the average power of the region other than the inner first region by 0.75 to 4.0 D.

**36.** The intraocular lens according to claim 33, wherein the first zone includes an outer transition region for connecting the inner first region to the outer first region.

**37.** The intraocular lens according to claim 32,

wherein the inner first region includes a constant power addition region including a power profile in which a positive constant power is added to the power profile Va for the first zone, and
when seen in the radial direction from the lens center O, a range in the radial direction of the constant power addition region is 33 to 67% of a range in the radial direction of the inner first region.

**38.** The intraocular lens according to claim 32, wherein the inner first region includes the lens center O.

**39.** The intraocular lens according to claim 33,

wherein the first zone includes an innermost region including the power profile Va for the first zone and including the lens center O, and
the inner first region surrounds the innermost region.

**40.** The intraocular lens according to claim 39, wherein the first zone includes an inner transition region for connecting the inner first region to the innermost region.

**41.** The intraocular lens according to claim 32, wherein an anterior surface, a posterior surface, or both of them of a lens body of the intraocular lens having two surfaces facing each other is or are aspherical.

**42.** The intraocular lens according to claim 32, wherein an anterior surface, a posterior surface, or both of them of a lens body of the intraocular lens having two surfaces facing each other includes or include a toric surface including a cylinder power for astigmatism correction for an aphakic patient.

**43.** The intraocular lens according to any one of claims 1 to 42, comprising at least any one of silicone, hydrophobic acrylic resin, hydrophilic acrylic resin, hydrogel, PMMA, PMMA copolymer, and collagen-containing HEMA (hydroxyethyl methacrylate) copolymer.

**44.** A method for designing an intraocular lens designing the intraocular lens according to any one of claims 1 to 42.

**45.** A method for manufacturing an intraocular lens manufacturing the intraocular lens designed by the method for designing an intraocular lens according to claim 44 by at least any one of lathe-turning, molding, and 3D printing.

# FIG. 1

Lens center O
First zone
First boundary
Second zone

# FIG. 2

100%
50%
10%
Va(I50)
Vb (O50)
Va (I10)
Vb (O10)
50%
100%
10%
Spheric
Zero aberration
Aspheric (SA -0.27)
New IOL
Dioptric power (D)
Radius (mm)
ral
rah
21
20
19
18
17
16
0
0.5
1
1.5
2
2.5
3

# FIG. 3

21
20
19
18
17
16
Power (D)
0
0.5
1
1.5
2
2.5
3
Radius (mm)
Inner power profile
Aspheric (SA -0.27)
I10
I35
I50
100%
50%
35%
10%

# FIG. 4

100%
50%
25%
10%
21
20
19
18
17
16
Power (D)
0
0.5
1
1.5
2
2.5
3
Radius (mm)
Outer power profile
Aspheric (SA -0.27)
O10
O25
O50

# FIG. 5

100%
25% (O25)
Inner region (I)
Outer region (O)
100%
35% (I35)
Power (D)
Radius (mm)
Aspheric (SA -0.27)
I35
O25
21
20
19
18
17
16
0
0.5
1
1.5
2
2.5
3


# FIG. 6

I35
O25
Power (D)
Radius (mm)
Aspheric (SA -0.27)
I35-O25
21
20
19
18
17
16
0
0.5
1
1.5
2
2.5
3

# FIG. 7

21
20
19
18
17
16
Power (D)
0
0.5
1
1.5
2
2.5
3
Radius (mm)
Aspheric (SA -0.27)
I10-O10
I40-O20
I50-O50


# FIG. 8

21
20
19
18
17
16
Power (D)
0
0.5
1
1.5
2
2.5
3
Radius (mm)
Aspheric (SA -0.27)
I10-O10
I50-O50

# FIG. 9

21
20
19
18
17
16
15
Power (D)
0
0.5
1
1.5
2
2.5
3
Radius (mm)
Aspheric (SA -0.27)
I10-O10
I50-O50
Non-percentage IOL

# FIG. 10

21
20
19
18
17
16
15
Power (D)
0
0.5
1
1.5
2
2.5
3
3.25
Radius (mm)
Inner region
Outer region
Additional outer region
Aspheric (SA -0.27)
I35-O25 (optic 6.5 mm)

# FIG. 11

I50
O50
Average absolute slope
0.46 D/mm
Average absolute slope
3.94 D/mm
Average absolute slope
2.36 D/mm
Average absolute slope
0.73 D/mm
Power (D)
Radius (mm)
Aspheric (SA -0.27)
I10-O10
I50-O50
I10
O10
21
20
19
18
17
16
0
0.5
1
1.5
2
2.5
3

# FIG. 12A

Mid region
Power (D)
Radius (mm)
Aspheric (SA -0.27)
I35-Mid1-O25
21
20
19
18
17
16
15
0
0.5
1
1.5
2
2.5
3

# FIG. 12B

Mid region
Power (D)
Radius (mm)
Aspheric (SA -0.27)
I35-Mid2-O25

# FIG. 12C

Mid region
Power (D)
Radius (mm)
Aspheric (SA -0.27)
I40-Mid3-O40

# FIG. 13

| | Curvature radius (mm) | Conic constant | Thickness (mm) | Refractive index |
|---|---|---|---|---|
| Anterior cornea | 7.8 | -0.07018 | 0.55 | 1.3771 |
| Posterior cornea | 6.5 | 0 | 4.00 | 1.336 |
| Pupil | Infinite | 0 | 0 | 1.336 |
| Anterior IOL (Aspheric -0.27 IOL) | Aspheric (center radius 16.973) | 0 | 0.626 | 1.520 |
| Posterior IOL (Aspheric -0.27 IOL) | -20.00 | 0 | 18.85 | 1.336 |
| Retina | — | — | — | — |

# FIG. 14

Total power (cornea and IOL)
Spheric
Zero aberration
Aspheric (SA -0.07)
Aspheric (SA -0.20)
I35-O25
Aspheric (SA -0.27)
Total power (D)
Radius (mm)
62.5
62
61.5
61
60.5
60
59.5
59
58.5
0
0.5
1
1.5
2
2.5
3

# FIG. 15

Total power (cornea and IOL)
Aspheric (SA -0.27)
I50-O50
I40-O20
I35-O25
I10-O10
Total power (D)
60
59.75
59.5
59.25
59
0
0.5
1
1.5
2
2.5
3
Radius (mm)

# FIG. 16

Total power (cornea and IOL)
Aspheric (SA -0.27)
I10-O10
I50-O50
Total power (D)
60
59.75
59.5
59.25
59
0
0.5
1
1.5
2
2.5
3
Radius (mm)

# FIG. 17A

Distance-MTF graph (On-axis)
Pupil size 3 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 17B

Distance-MTF graph (On-axis)
Pupil size 3.5 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 17C

Distance-MTF graph (On-axis)
Pupil size 4 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 17D

Distance-MTF graph (On-axis)
Pupil size 4.5 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 18A

Distance-MTF graph (Decenter 0.3 mm)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 18B

Distance-MTF graph (Decenter 0.3 mm)
Pupil size 3.5 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 18C

Distance-MTF graph (Decenter 0.3 mm)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0.8 1 1.25 1.5 1.75 2 2.5 3 4 5 6
Distance (m)


# FIG. 18D

Distance-MTF graph (Decenter 0.3 mm)
Pupil size 4.5 mm
Spheric
Zero aberration
Aspheric -0.27
I40-O20
I35-O25
MTF
1
0.9
0.8
0.7
0.6
0.5
0.4
0.3
0.2
0.1
0
0.8 1 1.25 1.5 1.75 2 2.5 3 4 5 6
Distance (m)

# FIG. 19A

Distance-MTF graph (Decenter 0.5 mm)
Pupil size 3 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 19B

Distance-MTF graph (Decenter 0.5 mm)
Pupil size 3.5 mm
Spheric
Zero aberration
Aspheric -0.27
I40-O20
I35-O25
MTF
0
0.1
0.2
0.3
0.4
0.5
0.6
0.7
0.8
0.9
1
0.8 1 1.25 1.5 1.75 2 2.5 3 4 5 6
Distance (m)

# FIG. 19C

Distance-MTF graph (Decenter 0.5 mm)
Pupil size 4 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 19D

Distance-MTF graph (Decenter 0.5 mm)
Pupil size 4.5 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 20A

Distance-MTF graph (Tilt 3°)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 20B

Distance-MTF graph (Tilt 3°)
Pupil size 3.5 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 20C

Distance-MTF graph (Tilt 3°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)


# FIG. 20D

Distance-MTF graph (Tilt 3°)
Pupil size 4.5 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 21A

Distance-MTF graph (Tilt 5°)
Pupil size 3 mm
Spheric
Zero aberration
Aspheric -0.27
I40-O20
I35-O25
MTF
Distance (m)
0
0.1
0.2
0.3
0.4
0.5
0.6
0.7
0.8
0.9
1
0.8
1
1.25
1.5
1.75
2
2.5
3
4
5
6


# FIG. 21B

Distance-MTF graph (Tilt 5°)
Pupil size 3.5 mm
Spheric
Zero aberration
Aspheric -0.27
I40-O20
I35-O25
MTF
Distance (m)
0
0.1
0.2
0.3
0.4
0.5
0.6
0.7
0.8
0.9
1
0.8
1
1.25
1.5
1.75
2
2.5
3
4
5
6

# FIG. 21C

Distance-MTF graph (Tilt 5°)
Pupil size 4 mm
Spheric
Zero aberration
Aspheric -0.27
I40-O20
I35-O25
MTF
0 0.1 0.2 0.3 0.4 0.5 0.6 0.7 0.8 0.9 1
0.8 1 1.25 1.5 1.75 2 2.5 3 4 5 6
Distance (m)

# FIG. 21D

Distance-MTF graph (Tilt 5°)
Pupil size 4.5 mm
Spheric
Zero aberration
Aspheric -0.27
I40-O20
I35-O25
MTF
0 0.1 0.2 0.3 0.4 0.5 0.6 0.7 0.8 0.9 1
0.8 1 1.25 1.5 1.75 2 2.5 3 4 5 6
Distance (m)

# FIG. 22A

Distance-MTF graph (Decenter 0.3 mm, tilt 3°)
Pupil size 3 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)


# FIG. 22B

Distance-MTF graph (Decenter 0.3 mm, tilt 3°)
Pupil size 3.5 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 22C

Distance-MTF graph (Decenter 0.3 mm, tilt 3°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 22D

Distance-MTF graph (Decenter 0.3 mm, tilt 3°)
Pupil size 4.5 mm
Spheric
Zero aberration
Aspheric -0.27
I40-O20
I35-O25
MTF
1
0.9
0.8
0.7
0.6
0.5
0.4
0.3
0.2
0.1
0
0.8
1
1.25
1.5
1.75
2
2.5
3
4
5
6
Distance (m)

# FIG. 23A

Distance-MTF graph (Decenter 0.4 mm, tilt 4°)
Pupil size 3 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 23B

Distance-MTF graph (Decenter 0.4 mm, tilt 4°)
Pupil size 3.5 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 23C

Distance-MTF graph (Decenter 0.4 mm, tilt 4°)
Pupil size 4 mm
Spheric
Zero aberration
Aspheric -0.27
I40-O20
I35-O25
MTF
Distance (m)
0
0.1
0.2
0.3
0.4
0.5
0.6
0.7
0.8
0.9
1
0.8
1
1.25
1.5
1.75
2
2.5
3
4
5
6

# FIG. 23D

Distance-MTF graph (Decenter 0.4 mm, tilt 4°)
Pupil size 4.5 mm
Spheric
Zero aberration
Aspheric -0.27
I40-O20
I35-O25
MTF
Distance (m)
0
0.1
0.2
0.3
0.4
0.5
0.6
0.7
0.8
0.9
1
0.8
1
1.25
1.5
1.75
2
2.5
3
4
5
6

# FIG. 24A

Distance-MTF graph (Decenter 0.5 mm, tilt 5°)
Pupil size 3 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 24B

Distance-MTF graph (Decenter 0.5 mm, tilt 5°)
Pupil size 3.5 mm
Spheric
Zero aberration
Aspheric -0.27
I40-O20
I35-O25
MTF
0
0.1
0.2
0.3
0.4
0.5
0.6
0.7
0.8
0.9
1
0.8
1
1.25
1.5
1.75
2
2.5
3
4
5
6
Distance (m)

# FIG. 24C

Distance-MTF graph (Decenter 0.5 mm, tilt 5°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0.8 1 1.25 1.5 1.75 2 2.5 3 4 5 6
Distance (m)

# FIG. 24D

Distance-MTF graph (Decenter 0.5 mm, tilt 5°)
Pupil size 4.5 mm
Spheric
Zero aberration
Aspheric -0.27
I40-O20
I35-O25
MTF
1
0.9
0.8
0.7
0.6
0.5
0.4
0.3
0.2
0.1
0
0.8 1 1.25 1.5 1.75 2 2.5 3 4 5 6
Distance (m)

# FIG. 25A

Decenter-MTF graph (Distance 2 m, tilt 0°)
Pupil size 3 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 25B

Decenter-MTF graph (Distance 2 m, tilt 3°)
Pupil size 3 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 25C

Decenter-MTF graph (Distance 2 m, tilt 5°)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)


# FIG. 25D

Decenter-MTF graph (Distance 2 m, tilt 0°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 25E

Decenter-MTF graph (Distance 2 m, tilt 3°)
Pupil size 4 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1


# FIG. 25F

Decenter-MTF graph (Distance 2 m, tilt 5°)
Pupil size 4 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 26A

Decenter-MTF graph (Distance 3 m, tilt 0°)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 26B

Decenter-MTF graph (Distance 3 m, tilt 3°)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 26C

Decenter-MTF graph (Distance 3 m, tilt 5°)
Pupil size 3 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 26D

Decenter-MTF graph (Distance 3 m, tilt 0°)
Pupil size 4 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 26E

Decenter-MTF graph (Distance 3 m, tilt 3°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 26F

Decenter-MTF graph (Distance 3 m, tilt 5°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 27A

Decenter-MTF graph (Distance 4 m, tilt 0°)
Pupil size 3 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1


# FIG. 27B

Decenter-MTF graph (Distance 4 m, tilt 3°)
Pupil size 3 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 27C

Decenter-MTF graph (Distance 4 m, tilt 5°)
Pupil size 3 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)


# FIG. 27D

Decenter-MTF graph (Distance 4 m, tilt 0°)
Pupil size 4 mm
Spheric
Zero aberration
Aspheric -0.27
I40-O20
I35-O25
MTF
1
0.9
0.8
0.7
0.6
0.5
0.4
0.3
0.2
0.1
0
0
0.1
0.2
0.3
0.4
0.5
0.6
0.7
Decenter (mm)

# FIG. 27E

Decenter-MTF graph (Distance 4 m, tilt 3°)
Pupil size 4 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 27F

Decenter-MTF graph (Distance 4 m, tilt 5°)
Pupil size 4 mm
Spheric
Zero aberration
Aspheric -0.27
I40-O20
I35-O25
MTF
Decenter (mm)
0
0.1
0.2
0.3
0.4
0.5
0.6
0.7
0.8
0.9
1

# FIG. 28A

Distance 2 m, pupil 3 mm, decenter 0 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 28B

Distance 2 m, pupil 3 mm, decenter 0.3 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 28C

Distance 2 m, pupil 3 mm, decenter 0.5 mm
Spheric
Zero aberration
Aspheric -0.27
I40-O20
I35-O25
MTF
Tilt (°)
1
0.9
0.8
0.7
0.6
0.5
0.4
0.3
0.2
0.1
0
0
1
2
3
4
5
6
7


# FIG. 28D

Distance 2 m, pupil 4 mm, decenter 0 mm
Spheric
Zero aberration
Aspheric -0.27
I40-O20
I35-O25
MTF
Tilt (°)
1
0.9
0.8
0.7
0.6
0.5
0.4
0.3
0.2
0.1
0
0
1
2
3
4
5
6
7

# FIG. 28E

Distance 2 m, pupil 4 mm, decenter 0.3 mm
Spheric
Zero aberration
Aspheric -0.27
I40-O20
I35-O25
MTF
1
0.9
0.8
0.7
0.6
0.5
0.4
0.3
0.2
0.1
0
0
1
2
3
4
5
6
7
Tilt (°)


# FIG. 28F

Distance 2 m, pupil 4 mm, decenter 0.5 mm
Spheric
Zero aberration
Aspheric -0.27
I40-O20
I35-O25
MTF
1
0.9
0.8
0.7
0.6
0.5
0.4
0.3
0.2
0.1
0
0
1
2
3
4
5
6
7
Tilt (°)

# FIG. 29A

Distance 3 m, pupil 3 mm, decenter 0 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 29B

Distance 3 m, pupil 3 mm, decenter 0.3 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 29C

Distance 3 m, pupil 3 mm, decenter 0.5 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7


# FIG. 29D

Distance 3 m, pupil 4 mm, decenter 0 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 29E

Distance 3 m, pupil 4 mm, decenter 0.3 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 29F

Distance 3 m, pupil 4 mm, decenter 0.5 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 30A

Distance 4 m, pupil 3 mm, decenter 0 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 30B

Distance 4 m, pupil 3 mm, decenter 0.3 mm
Spheric
Zero aberration
Aspheric -0.27
I40-O20
I35-O25
MTF
Tilt (°)
0
0.1
0.2
0.3
0.4
0.5
0.6
0.7
0.8
0.9
1
0
1
2
3
4
5
6
7

# FIG. 30C

Distance 4 m, pupil 3 mm, decenter 0.5 mm
MTF
Tilt (°)
Spheric
Zero aberration
Aspheric -0.27
I40-O20
I35-O25
0
0.1
0.2
0.3
0.4
0.5
0.6
0.7
0.8
0.9
1
0
1
2
3
4
5
6
7

# FIG. 30D

Distance 4 m, pupil 4 mm, decenter 0 mm
MTF
Tilt (°)
Spheric
Zero aberration
Aspheric -0.27
I40-O20
I35-O25
0
0.1
0.2
0.3
0.4
0.5
0.6
0.7
0.8
0.9
1
0
1
2
3
4
5
6
7

# FIG. 30E

Distance 4 m, pupil 4 mm, decenter 0.3 mm
MTF
Tilt (°)
Spheric
Zero aberration
Aspheric -0.27
I40-O20
I35-O25
0
0.1
0.2
0.3
0.4
0.5
0.6
0.7
0.8
0.9
1
0
1
2
3
4
5
6
7


# FIG. 30F

Distance 4 m, pupil 4 mm, decenter 0.5 mm
MTF
Tilt (°)
Spheric
Zero aberration
Aspheric -0.27
I40-O20
I35-O25
0
0.1
0.2
0.3
0.4
0.5
0.6
0.7
0.8
0.9
1
0
1
2
3
4
5
6
7

# FIG. 31A

Embodiment 5A
Inner first zone
Outer first zone
First zone

# FIG. 31B

Embodiment 5B
Inner first zone
Outer first zone
Innermost zone
First zone

# FIG. 32A

First zone
Inner region (I)
Second zone
Outer region (O)
Inner first region
Outer first region
Constant power addition region
Positive power deviation region
Power (D)
22
21
20
19
18
17
16
0
0.5
1
1.5
2
2.5
3
Radius (mm)
Aspheric (SA -0.27)
I40-O20
Embodiment 5A

# FIG. 32B

Inner region (I)
Outer region (O)
Inner first region
Innermost region
Outer first region
Constant power addition region
Positive power deviation region
Power (D)
Radius (mm)
22
21
20
19
18
17
16
0
0.5
1
1.5
2
2.5
3
Aspheric (SA -0.27)
I40-O20
Embodiment 5B

# FIG. 33A

Embodiment 5A
Outer Transition region
Power (D)
Radius (mm)
Aspheric (SA -0.27)
I40-O20
Embodiment 5A
22
21
20
19
18
17
16
0
0.5
1
1.5
2
2.5
3

# FIG. 33B

Embodiment 5B
Inner transition region
Outer transition region
Power (D)
Radius (mm)
22
21
20
19
18
17
16
0
0.5
1
1.5
2
2.5
3
Aspheric (SA -0.27)
I40-O20
Embodiment 5B

# FIG. 34

Aspheric (SA -0.27)
I40-O20
Embodiment 5A
Constant addition power (constant power difference)
Range of lens radius having constant addition power ($R_{Constant}$)
Total range of lens radius of add power sub-region ($R_{Total}$)
Power (D)
Radius (mm)
22
21
20
19
18
17
16
0
0.5
1
1.5
2
2.5
3

**Condition:** $R_{Constant} \geq (33\%\ R_{Total})$ and $R_{Constant} \leq (67\%\ R_{Total})$

## INTERNATIONAL SEARCH REPORT

International application No.

**PCT/JP2024/040261**

### A. CLASSIFICATION OF SUBJECT MATTER

***A61F 2/16***(2006.01)i; ***G02C 7/00***(2006.01)i; ***G02C 7/02***(2006.01)i
FI: A61F2/16; G02C7/02; G02C7/00

According to International Patent Classification (IPC) or to both national classification and IPC

### B. FIELDS SEARCHED

Minimum documentation searched (classification system followed by classification symbols)

A61F2/16; G02C7/00; G02C7/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2009-536052 A (CARL ZEISS MEDITEC AG) 08 October 2009 (2009-10-08) paragraphs [0052]-[0053], fig. 6 | 1-45 |
| A | CN 109363802 A (WUXI VISION PRO LTD.) 22 February 2019 (2019-02-22) entire text, all drawings | 1-45 |
| A | WO 2021/111821 A1 (HOYA CORPORATION) 10 June 2021 (2021-06-10) entire text, all drawings | 1-45 |
| A | JP 2007-330478 A (HOYA CORPORATION) 27 December 2007 (2007-12-27) entire text, all drawings | 1-45 |
| A | US 2010/0234943 A1 (PORTNEY, Valdemar) 16 September 2010 (2010-09-16) entire text, all drawings | 1-45 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 December 2024** | **07 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/040261**

| Patent document cited in search report | Publication date (day/month/year) | Patent family member(s) | Publication date (day/month/year) |
|---|---|---|---|
| JP 2009-536052 A | 08 October 2009 | US 2009/0125105 A1<br>paragraphs [0077]-[00578], fig. 6<br>WO 2007/128423 A1<br>DE 102006021521 A1<br>CN 101437468 A<br>KR 10-2009-0020588 A | |
| CN 109363802 A | 22 February 2019 | (Family: none) | |
| WO 2021/111821 A1 | 10 June 2021 | US 2022/0401210 A1<br>entire text, all drawings<br>EP 4070761 A1 | |
| JP 2007-330478 A | 27 December 2007 | US 2009/0270984 A1<br>entire text, all drawings<br>WO 2007/145082 A1<br>EP 2033596 A1 | |
| US 2010/0234943 A1 | 16 September 2010 | WO 2010/104530 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007330478 A
- US 20050203619 A
- WO 2004090611 A
- US 20040088050 A
- WO 2006060477 A
- WO 2007128423 A
- WO 2009153873 A
- WO 2008078804 A